(19) Europäisches Patentamt

European Patent Office

Office européen des brevets

(11) **EP 0 957 966 B1**

(12) **EUROPEAN PATENT SPECIFICATION**

(45) Date of publication and mention
of the grant of the patent:
**07.09.2005 Bulletin 2005/36**

(21) Application number: **96921334.7**

(22) Date of filing: **06.06.1996**

(51) Int Cl.⁷: **A61M 25/00**

(86) International application number:
**PCT/US1996/009274**

(87) International publication number:
**WO 1997/025090 (17.07.1997 Gazette 1997/31)**

(54) **ROTATE-TO-ADVANCE CATHETER SYSTEM**

DURCH DREHUNG VORSCHIEBBARES KATHETERSYSTEM

SYSTEME DE CATHETER A AVANCE PAR ROTATION

(84) Designated Contracting States:
**DE FR GB IT**

(30) Priority: **11.01.1996 US 585040**
**26.02.1996 US 12276 P**

(43) Date of publication of application:
**24.11.1999 Bulletin 1999/47**

(73) Proprietor: **Frassica, James J.**
**Chelmsford, MA 01824 (US)**

(72) Inventor: **Frassica, James J.**
**Chelmsford, MA 01824 (US)**

(74) Representative: **Thomson, Paul Anthony**
**Potts, Kerr & Co.**
**15, Hamilton Square**
**Birkenhead**
**Merseyside CH41 6BR (GB)**

(56) References cited:
**DE-A- 2 311 807**   **DE-U- 9 203 077**
**US-A- 1 644 919**   **US-A- 1 888 349**
**US-A- 5 313 934**

**Description**

BACKGROUND OF THE INVENTION

TECHNICAL FIELD OF THE INVENTION

This invention most generally relates to the apparatus and methods of catheterization and related treatments of the genito-urinary and gastro-intestinal passages of mammals. More particularly, the invention relates to catheters, dilators, occluders, stents, suprapubic catheters, camera introducers and related medical devices subject to being proximally propelled and directed for advancement and control in mammalian genito-urinary and gastro-intestinal passages.

DESCRIPTION OF THE PRIOR ART

In most mammals, mucous membranes line all those passages by which the internal parts communicate with the exterior, and are continuous with the skin at the various orifices of the surface of the body. They are soft and velvety, and very vascular, and their surface is coated over by their secretion, mucus, which is of a tenacious consistence, and serves to protect them from the foreign substances introduced into the body with which they are brought in contact.

They are described as lining the two tracts - the genito-urinary and the gastro-intestinal; and all, or almost all, mucous membranes may be classed as belonging to and continuous with the one or the other of these tracts. Catheterization of any of these similar bodily passages may at times be useful or necessary.

Urinary outlet problems most likely have been around for as long as humans. History has the ancient Chinese using onion stalks to relieve people of acute urinary retention. Literature refers to such problems as far back as 206 B.C., more than 2000 years ago. Romans used catheters, first invented by Erasistratus, a Greek doctor in the third century B.C. Roman catheters were fine tubes made of bronze. The Roman gynecologist Soranus describes how catheters could be used to push stones out of the way and back into the cavity of the bladder, and thus restore urine flow. Excavations in Pompeii unearthed several bronze catheters. These instruments were well constructed but relatively simple and showed that designs changed little from the period 79 AD until 1700 A.D.

However, during the 17th and 18th centuries catheter construction became more complex with an intensified search for an appropriate substance that would be at once flexible, non-irritating and functional. England, France, and the U.S.A. had individuals and companies deeply involved with urinary catheters during this period. Many variations were produced but they all caused much stress on the patient when these rigid devices were pushed into the urethra. The first practical breakthrough was by the French using gum elastic catheters - a catheter that would bend better in the urethral channel and not scour the mucosa so much in the process.

Charles Goodyear improved upon what the French produced when he successfully vulcanized crude rubber. The problem of manufacturing an instrument which was both sufficiently rigid to enable it to be pushed through the urethra into the bladder and yet flexible enough to negotiate the path, had at last reached the point of practicality, not withstanding its shortcomings. At that time, and still to this day, a functional urethral catheter is defined as one that is flexible enough to negotiate the bends and stable enough to push through the length of the urethral passage.

The French urologist J.J. Cazenave, with the hopes that his country would regain leadership in the catheter field, dedicated 25-30 years of his life improving the flexible durable catheter. This was in the late 1800's and his catheter, made of decalcified ivory, was a dated device but shows the consistency of the state of the art wherein catheters are pushed into and negotiated along the urethral passage toward the bladder.

During the past 300 years or so, intensified development efforts were stimulated by professional pride, national pride and financial rewards. These efforts yielded many improvements, such as changes to size, curve shape, materials of construction, smoothness, lubricants, coatings, combinations of materials, physical properties, chemical properties and more, yet all subscribed to the basic principle of external push-to-advance.

The catheters of the prior art are large and stiff, difficult and uncomfortable to administer, and uncomfortable to wear for extended periods. There is a degree of skill, tolerance and patience required that takes much time, training and practice to learn. The difficulty, discomfort, risk of injury and infection, inhibition and inconvenience of the methods and tools of the known art results in the deprivation for many patients of the freedom to work, play and travel as do unaffected people.

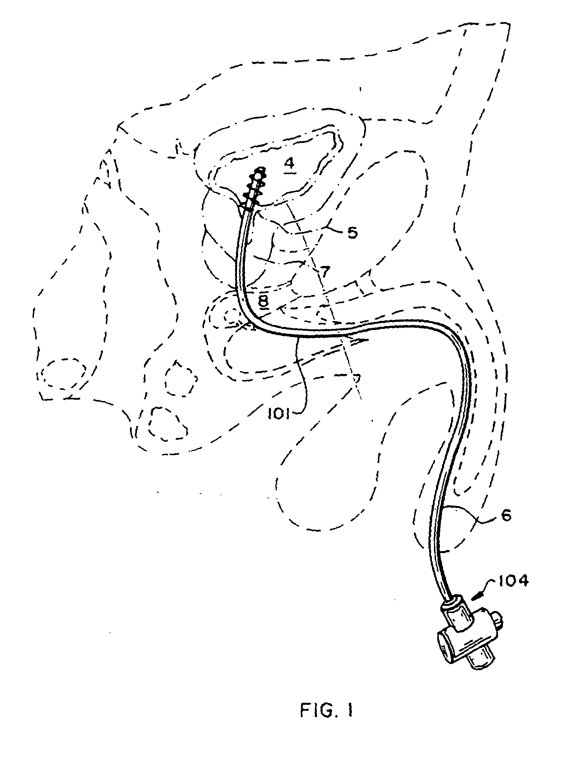

The anatomy of the adult male urinary tract, as illustrated in Fig. 1, has a bladder 004 where urine is collected prior to exiting the body via the urethra 006. The bladder 004 converges into the urethra 006 at a muscular exit called the bladder neck 005. Approximately the first one inch of the urethra lies within the prostate 007, which is a chestnut-sized gland. The next approximately half inch passes through the external sphincter 008, which is the muscular flow valve that controls the release of urine. The remaining six inches of the urethra lie in a spongy zone, exiting the body at the meatus 009.

The normal process of emptying the bladder can be interrupted by two causes. One is bladder outlet obstruction and the other is failure of the nerves linking the bladder to the brain. The most frequent cause of bladder outlet obstruction in males is enlargement of the prostate gland by hypertrophy or hyperplasia. In older males, it is not uncommon for a progressive enlargement of the prostate to constrict the prostate urethra. This condition, known as benign prostatic hyperplasia (BPH), can cause a variety of obstructive symptoms, including urinary hesitancy, straining to void, decreased size and force of the urinary stream and in extreme cases, complete urinary retention possibly leading to renal failure.

The most common surgical intervention for BPH, transurethral resection of the prostate, or TURP, has a lengthy recovery period of up to one year, and presents a high operative risk for complications such as sexual dysfunction. Up to 10% of those subjected to such surgery are left with mild to moderate stress incontinence. Approximately 400,000 patients in the United States and approximately 500,000 patients internationally were diagnosed in 1994 with BPH or cancer-induced bladder outlet obstructions that were sufficiently severe to warrant TURP or alternative surgery, according to industry sources.

Because of the high costs, medical risks and quality of life compromises associated with TURP, new technologies have begun to challenge TURP's position as the standard treatment for severe BPH. Recently, the U.S. Food and Drug Administration approved two drugs, tera zosin hydrochloride and rinasteride, to treat BPH. These drugs generally do not improve symptoms for six to nine months after treatment begins, and are not without side effects.

Urethral strictures are another cause of outlet obstruction, often due to fibrous tissue growth resulting from reaction to catheters or cystoscopes or from injury, birth defects or disease, and are commonly treated by urethral dilation, catheterization or surgery. Men with urethral strictures also experience a limited ability to urinate, which may cause extreme discomfort and, if left untreated may cause complications that necessitate catheterization. Approximately 50,000 patients in the United States were diagnosed with recurrent urethral strictures in 1994, according to industry sources. The inventor estimates that approximately 75,000 additional patients were diagnosed internationally.

Women suffer from urinary incontinence far more often than men and at an younger age primarily because of the stress associated with pregnancy and childbirth, the shorter length of the female urethra, and the absence of a prostate. The U.S. Department of Health and Human Services (HHS) estimates that the involuntary loss of urine affects approximately 10 million Americans of which 8.5 million are women. Seven million of these women are non-institutionalized, or community-dwelling.

For women between the ages of 15 and 64, the prevalence of urinary incontinence is estimated to range from 10 to 25 percent of the population. For non-institutionalized persons over the age of 60, the prevalence of urinary incontinence ranges from 15 to 30 percent, with the prevalence in women twice that of men.

The involuntary loss of urine can be caused by a variety of anatomical and physiological factors. The type and cause of urinary incontinence is important to how the condition is treated and managed. The two broad categories of urinary incontinence are urge and stress incontinence. Some people suffer from what is termed mixed incontinence or a combination of stress and urge incontinence.

Urge incontinence is the involuntary loss of urine associated with an abrupt and strong desire to void. In most cases, urge incontinence is caused by involuntary detrusor (the smooth muscle in the wall of the bladder) contractions or over-activity. For many people, urge incontinence can be satisfactorily managed with pharmaceuticals.

The more frequently occurring stress incontinence is the involuntary loss of urine caused by movement or activity that increases abdominal pressure. The most common cause of stress incontinence is hypermobility or significant displacement of the urethra and bladder neck during exertion. A less frequent cause of stress incontinence is intrinsic urethral sphincter deficiency (ISD), a condition in which the sphincter is unable to generate enough resistance to retain urine in the bladder.

Females, and males with no benign prostatic hyperplasia condition, might also have the inability to empty their bladder because of the nerves linking the bladder to the brain. This condition is known as neuropathic bladder, may occur in a wide variety of conditions which include spina bifida, multiple sclerosis, spinal injury, slipped disc and diabetes. When these and other problems prevent the bladder from effectively controlling urine there are a number of treatment options. They are catheters, dilators, occluders, and stents.

During continuous catheterization an indwelling catheter is retained in the bladder by a water filled balloon. It drains urine continuously from the bladder via a connecting tube into a bag which is attached to the leg or bed. The bag has a tap so that the urine can be emptied at intervals. The catheter is usually inserted by a doctor or nurse and changed about every four to six weeks. But difficulty in placement has always been inherent in this design. This is due to the traditional "push to advance" technology, which necessitates a stiff thick-walled catheter to traverse the delicate mucosal lined urethra.

Often the French (unit of measurement) size of the catheter is dictated by the need for stiffness to insert rather than the lumen size to pass urine. A 14 French or smaller Foley is rarely used because catheters of this size lack the column strength needed to push the full length of the urethra into the bladder. The larger French Foley catheters are painful to place, uncomfortable when indwelling, and require a highly skilled care provider to insert.

During intermittent catheterization a simple catheter made of plastic, rubber, or metal is inserted by the patient or a helper for just long enough to empty the bladder completely, which is typically about one minute. These tubes are usually smaller in diameter and stiffer than an indwelling catheter of the same size. This stiffness can make catheterization difficult in men because the urethra is long and has an acute bend within the prostate. When the external sphincter is reached the sphincter muscle will contract making passage difficult. Most patients learn to catheterize themselves and thereby gain a large degree of independence. This process is repeated about every 3-4 hours during the day and occasionally as needed at night.

Intermittent catheterization is mainly used by people who are incontinent due to neuropathic bladder. Intermittent catheterization may also be utilized by people who cannot empty the bladder because the bladder muscle is weak and does not contract properly. In some patients, an alternate apparatus and method used to maintain long term drainage of the bladder is the use of a suprapubic tube.

Suprapubic catheterization of the bladder is performed via transabdominal puncture which enters the body above the pubic arch and is directed into the bladder using ultrasound or fluoroscopy to guide the trocar introducer and suprapubic catheter. The needle introducer is then removed when proper catheter placement within the bladder is confirmed, leaving the drainage catheter in place.

Long term drainage may require the fixation of the catheter at the skin using standard adhesive based interface components to address mechanical fixation, inflection control, and skin compatibility. The distal end of the catheter is commonly contained within the bladder by inflated balloon, winged-shaped tip configurations which expand within the bladder, or preshaped curved catheter tips which curl to their original J-shape when stiffening wire is removed from the catheter lumen.

A problem with this form of distal end emplacement is that it is only unidirectional; that is, it only resists the inadvertent pulling out of the tip of the catheter from the wall of the bladder, while allowing the catheter to freely pass further into the bladder, and back out up to the point of the containment structure. This continuing catheter motion in and out of the bladder may irritate tissue and cause infection or other difficulty at the bladder-catheter interface. Urine is especially irritating to most parts of the human body that are outside the urinary tract.

Dilation is accomplished by pushing successively larger urethral dilation tubes through the urethra to increase the size of the lumen, a procedure which is painful and traumatic to the patient. Surgical treatment of strictures involves surgical risks as well as complications, including infection, bleeding and restenosis, which requires further treatment.

With the exception of balloon catheters, the current art of dilators has also changed little over the passage of time. A shaft with an increasing taper, bulbus structure, or enlarged end is pushed from without the passage to advance the tool through the restricted passage, thus forcing by longitudinally-applied pressure the lateral expansion of the passage walls. This push-to-advance method necessitates a stiff shaft which has all the same liabilities as traditional catheters. Catheters inherently provide a degree of this dilatorial function to the extent that the passage is opened sufficiently to accommodate the catheter.

Occluders are used in some cases to control incontinence. Occluders of the prior art are constructed and applied with the same push-to-advance concept as catheters and dilators described above, with the same liabilities. The basic occluder is a bulb or plug on a shaft which is inserted within a passageway to stop or prevent the normal flow of materials through the passageway, or driven all the way into the bladder, for example, and allowed to seat as a plug at the neck of the urethra to prevent the flow of urine from the bladder.

A stent is a tubular metalic mesh device that is implanted to open and support a stricture to allow for urine flow. The stent body is between 3.5cm and 6.5cm in length depending on the anatomy, and is expandable by design to anchor in place. The stent being a mesh has openings that allow the tissue to grow through the wall making removal difficult and causing encrustation that reduces urine flow.

Among the prior art, the 1927 U.S. patent # 1,644,919 by Oscar Hayes discloses a catheter with a spiral rib, which is described as able to worm it's way in with only a slight rotary action. Hayes discloses a spiral structure described as easing the insertion of the device, clearly relying to a substantial degree on a push-to-advance methodology. Further, Hayes teaches that his device is then purposely withdrawn without rotation, intentionally scrubbing and scouring the passageway walls in a fashion suitable to his purpose, but totally contradictory to the goal of the instant invention. The long thread pitch and low thread height illustrated in the Hayes figures is too general in description and not suggestive of a device with the necessary thread height, low ratio of thread pitch to circumference, and limited length of helix necessary to effectuate purely rotate-to-advance pulling and anchoring power. The stated purpose and method of use of Hayes instead teaches away from the concept of the invention.

U.S. Patent 207,932, by W.I. Alvord disclosed in 1878 a metal spiral with a tapered end as a component to a surgical dilator which is described as applied by giving it a gentle rotary motion, the spiral causing the dilator to advance slowly into position. Alvord's dilator is by definition a tool for forcing a passageway to expand, necessarily placing his metal wire spiral with unyielding pressure against the wall of the passageway and likely causing pain and discomfort. The many loose coils and turns of the Alvord spiral have a non-uniform pitch and no suggestion of a reason for limiting the length, height, or pitch of the threads. As stated with regard to Hayes, the general description of the spiral feature is devoid of the specifics necessary to suggest or accomplish the purpose and effect of the instant invention.

The current device used for inspection and treatment of the GI (gastro-intestinal) tract is a flexible Endoscope. This device takes a high level of skill to use, is difficult to maneuver and can be very painful for the patient, due to the basic push-to-advance design that has not changed since the device was invented in the early 1960s. The distal tip of the endoscope has the following parts:

    1. a channel opening for suction and passage of accessories,
    2. the light guide lens to distribute light from the fiberoptic bundle to illuminate the visual field,
    3. the objective lens to focus an image of the mucosa onto the face of the image bundle and transmit it back to the eye piece,
    4. an air/water jet, which supplies air to inflate the organ being observed, and water to clean off the image lens.

The Bending Section is the distal end of the tube, ranging from approx.8-15cm long, which can articulate to steer the scope as it is pushed inward and is controlled by a cable mechanism that is connected to control knobs on the proximal handle.

The Insertion Tube, which makes up the rest of the 60-150cm length, is not capable of controlled deflection. It has a tailored bending flexibility and torque transmission which is of major importance in endoscope design. Most instruments have two-stage bending stiffness, i.e., the distal portion of the insertion tube is more flexible than the proximal portion. The Flexibility of each portion of the insertion tube requires extensive clinical testing to ensure that the endoscope handles easily and produces a minimum of patient discomfort.

The colon is a tubular organ which runs from the cecum in the right lower quadrant to the rectum . It is widest in the cecum and ascending colon and gradually narrows as one approaches the rectum. The colon is divided into the following sections:

    a. the cecum; the ascending colon, which runs cephalad (towards the head) from the cecum to the hepatic flexure;
    b. the transverse colon, which runs from the hepatic flexure tin the upper quadrant of the splenic flexure in the left upper quadrant;
    c. the descending colon, which runs caudad(toward the feet) from the splenic flexure to the left lower quadrant;
    d. the sigmoid colon, which runs from the left lower quadrant to the rectosigmoid junction; and
    e. the rectum, which extends down to the anal canal.

The inner layer of circular muscle is present throughout the colon. The outer longitudinal muscle in the wall of the colon is fused into three bands, the teniae coli. These bands start at the base of the appendix and run in the wall of the colon down to the rectum where they diffuse into the muscular coat. The three teniae cause the colon to have a triangular appearance endoscopically; this is especially prominent in the ascending and transverse colon. The haustra are outpouchings of the colon, separated by folds. In the descending colon the endoscopic appearance is often tubular.

Most experienced colonoscopists use similar endoscopic techniques. Air is introduced to inflate the colon, but as little as possible to prevent over distension. The pressure on the device is gentle to avoid stretching the colonic wall or mesentery (the connective tissue that holds the colon like a fan) which can cause pain, a vagal episode, or a perforation. The lumen is kept in view at all times; little or none of the examination is performed blindly, because you are pushing a stiff instrument.

A variety of in and out maneuvers are used to "accordion" the colon on the colonoscope, keeping the colonoscope free of loops as possible. In the difficult colon, special maneuvers such as the creating of an alpha loop in the sigmoid colon are used to pass the sharply angulated sigmoid/descending colon junction. This maneuver may require fluoroscopic guidance and training in the technique.

The colonoscope is advanced to the cecum under direct vision. The detailed examination of the mucosa is usually performed as the colonoscope is slowly removed from the cecum.

To inspect the whole length of the large intestine requires a highly skilled practitioner, which makes the procedure costly. Even still the procedure can be very painful for the patient, making sedation necessary. This is due to the inherent deficiencies in the "push-to-advance" design.

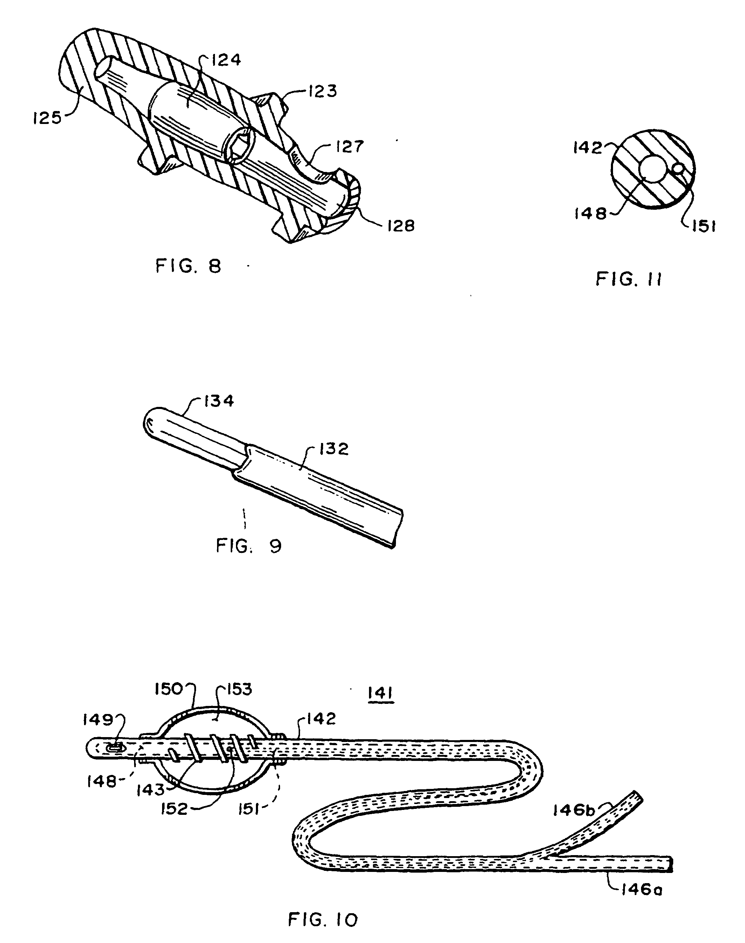

US 5,313,934 discloses a hollow tubular elongated member concentrically mounted to a surgical viewing instrument. In one embodiment (shown in Fig. 8) the tube 90 is flexible and includes a rib 88 defining a helical channel on the outer surface of the tube for delivering fluid to the distal end of the tube for cleaning the lens of the viewing instrument.

US 1,888,349 discloses a catheter having a spirally grooved peripheral region 12 for holding a salve-like material which may contain a medicament and which also serves as a lubricant.

In summary, there are problems in making present push-in-catheters, dilators, and occluders stiff enough for penetration and flexible enough to make the turns without undue risk of trauma to the wall of the passageway when being pushed in; and once installed, comfortable enough to wear for an extended period. And the problem with stent encrustation and removal are well known. Self-administration is inhibited by all of the shortcomings of the present art, and further injury, infection and discomfort may result from the resulting improper self-care. The problems with colonoscopy have been previously described.

The long history of push-in urinary catheters/dilators and occluders the longitudinal folds of the walls of the urethra and ureter have fostered a here-to-for untested assumption that any other approach would somehow be unworkable and possible damage or scour the mucosa further, or otherwise cause pain or distress to the subject. The soft, moist, pliant wall quite simply does not suggest a capability for providing a longitudinal grip on a threaded or helical device while allowing the sliding passage of the threads without incurring damage, but rather suggests or leads back to the traditional push-in tools and methodology.

SUMMARY OF THE INVENTION

According to the present invention there is provided a threaded catheter as claimed in claim 1, a threaded dilator as claimed in claim 5, a threaded stent as claimed in claim 6, a threaded suprapubic catheter for drainage of a genito-urinary organ as claimed in claim 8 and a threaded camera introducer system for accessing the gastro-intestinal tract as claimed in claim 12.

For the purposes of this disclosure, including the appended claims, the terms "distal", "distally", and "distal end", as they relate to the devices and methods described herein, refer to the end of the device *further* from or in the direction *away* from a practitioner who might be applying the device or method to the subject. Stated conversely, the terms refer to the end of the device closer to or in the direction towards the subject's interior, or in plain language, the front end of the device or direction in which the device is moved to be advanced into the subject's body.

The terms "proximal", "proximally", and "proximal end", as they relate to the devices and methods described herein, refer to the end of the device closer to or in the direction towards the practitioner who might be applying the device or method. Stated conversely, these terms refer to the end of the device further from or in the direction away from the subject's interior, or in plain language, the back end or external end or direction in which the device is moved to be retracted out of the subject's body.

Objects of the invention include providing and employing screw-based means for rotational advancement and anchoring of catheters, probes, occluders, stents, and dilators into genito-urinary and gastro-intestinal passageways such as the urethra, ureter, esophagus and fallopian tube, and for the emplacement of suprapubic catheters for draining genito-urinary organs such as the bladder, whereby the subject device is drawn through the passage by the longitudinal pull of a helix on the walls of the passage or organ as the distal end of the device is rotated. This technology is a radical departure from the 4000 year old traditional "push to advance" methodology previously discussed.

Flexible, thin wall indwelling and intermittent catheters and related devices and delivery stylets, made possible by this form of emplacement, are less traumatic and easier for the medical practitioner or patient to use. Once placed, the device is anchored by the radial force and friction of the helix, preventing longitudinal migration due to body movement or fluid flow. This technology fosters reduced costs for patent care, improved clinical outcomes and enhanced patient quality of life.

The male continence catheter of the invention, indicated for bladder outlet obstructions, is intended for BPH patients who are not able to, or choose not to undergo TURP. This embodiment of the invention allows the urethra in the area of the prostate to remain open. The catheter is inserted in the urethra by the user on a daily basis and taken out prior to sleeping. This catheter offers an effective option to surgery. It gives the patient the ability to regain control with a disposable catheter that is easily inserted into the urethra and worn during the day, and then removed and disposed of before retiring for the night.

The prostate stent of the invention, indicated for bladder outlet obstructions, is intended for BPH patients who are not able to, or choose no to undergo TURP. The prostate stent keeps the urethra open in the area of the prostate. The stent body may be between 3.5cm and 6.5cm in length depending on the anatomy, and has a helical element on the outer diameter of the body to advance and retain the stent. The sidewall of the stent may have a reinforcement means to prevent collapsing due to prostate pressure. The stent can be inserted in the urethra under fluoroscopy, using a detachable flexible stylet which keys into the proximal end of the stent body, and may be inserted in an outpatient procedure using topical anesthesia.

The urethral stent of the invention may be implanted in the male urethra as an alternative to dilation, catheterization and surgery. It keeps the urethra open by providing support under the stricture, thereby permitting passage of urine. The stent body may be between 3.5cm and 6.5cm in length depending on the anatomy, and has a tapered helix on the outer diameter of the body to advance and retain the stent. The sidewall of the stent may be reinforced to prevent collapsing due to prostate pressure.

The prostate and urethral stents of the invention may be inserted in the urethra using a detachable flexible stylet which keys into the proximal end of the stent body, and may be inserted in an outpatient procedure using topical anesthesia. The stent is not susceptible to being incorporated by the urethral mucosa in a manner preventing rotation, thereby permitting a lengthy period of emplacement and subsequent removal by the same rotational technique. The stent may also have a sufficiently large internal diameter, or lumen, to permit cystoscopies, thereby allowing examination of the bladder without removing the stent.

The methodology of this catheter uses the core helix technology to enable easy insertion and retaining of the device. The catheter shaft may have a short tapered *semi-pneumatic* helix on the distal 2cm (bladder end) of the shaft, and a drainage port at the distal end of the shaft. At the proximal (external end) there may be a flow valve which can be depressed to empty the bladder. It may be produced as a sterile, single-use, disposable item that can be used intermittently when the patient desires.

The patient simply inserts the catheter into the urethral opening and rotates the shaft to advance the catheter into the bladder. This can be done in the morning in the convenience of home. When the user needs to urinate, the valve end of the flexible shaft may be exposed through the clothing and the valve opened to empty the bladder. Since the device is not removed and reinserted after each voiding the risk of infection is reduced. At the end of the day the catheter is easily removed and disposed.

Another embodiment of the catheter of the invention provides a female Stress UI sufferer with lifestyle benefits that greatly outperform absorbent products intended to manage this condition. The catheter uses the core helix technology to enable easy insertion and retaining of the device. The catheter shaft may have a short tapered semi-pneumatic helix on the distal 2cm (bladder end) of the shaft, and a drainage port at the distal end of the shaft There may be a flange 3.5 cm from the distal end which seats in the urethral opening to position the catheter. At the distal (external end) there may be a flow valve which is opened to empty the bladder. It may be supplied as a sterile, single-use, disposable item that can be used intermittently when the patient desires.

The female patient simply inserts the catheter into the urethral opening and rotates the shaft to advance the catheter into the bladder. This can be done in the morning in the convenience of the patient's own home. There are no applicators to attach or dispose of When the user needs to urinate, the valve end of the flexible shaft is extended through the clothing or otherwise exposed, and the valve is opened to empty the bladder.

Since the device is not removed and reinserted into the urethra after each voiding the risk of infection is greatly reduced. Another benefit of this system is the user can void while standing, which may be of concern when using unsanitary public restrooms. At the end of the day the catheter is easily removed and disposed of.

The invention eliminates the push-to-advance characteristic of the traditional Foley Catheter by utilizing rotational advancement so that the catheter is drawn through the urethra by the longitudinal pull of the helix on the walls of the passage as the distal end of the device is rotated.

In this embodiment, the helix is located on the shaft under the balloon and disappears when the balloon is inflated. The flexible reinforced shaft need be only about half the wall thickness of conventional Foley Catheters, which means a smaller OD catheter can be used. The helix advances the shaft and dilates the urethra as the catheter is inserted. Once the bladder is reached the balloon is inflated with sterile water and the helix is engulfed by the balloon. The process is then reversed to remove the catheter.

Helically-adapted dilators and occluders of the invention are likewise rotatingly advanced and retracted; the helical element performing a dilatory function to some degree. Dilators of respectively larger diameters may be used to achieve a gradually more pronounced effect. The rotational advancement means may be combined with the push-to-advance methodology in any of these devices. In a dilator, for example, a helically equipped leader shaft extending distally of the bulbous portion of the device rotatingly advances the device up to the point that the helix passes out of the interior end of the passage, the remainder of the leader shaft then providing a guide wire that leads the bulb through the remainder of the passageway when the dilator is pushed from the proximal end.

The adaptation of the invention to suprapubic catheters used in a classic transabdominal puncture for the drainage of the bladder or other genito-urinary organs, permits the helix on the distal end of the catheter to be emplaced in the wall of the organ far enough so that the helical vane extends from both sides of the organ wall, so that the longitudinal sliding motion of the catheter into and out of the organ is inhibited by the helical vane. This reduces a source of irritation and associated complications at the organ wall entry point.

The helically-adapted suprapubic catheter may be placed in the organ using ultrasound or fluoroscopy to visualize placement, by rotatingly advancing the catheter over a guidewire leading to the organ; the guidewire having been installed through a tubular access created by using a cannula and trocar to reach the organ, the trocar and the cannula having been successively removed.

Any embodiment of the invention may be radiopaque, or have radiopaque features, markers or other components, permitting the use of fluoroscopy to monitor emplacement or removal of the device, or even the rotational orientation and rotational movement.

The thread element may be solid, hollow, or fluid filled. Embodiments or elements of the invention may be fabricated, molded, wound, extruded or otherwise constructed of nontoxic, non-corrosive materials or combinations of materials that are otherwise tolerant of bodily fluids and durable when implanted in vivo. Such materials may include but are not limited to polyurethane, medical grade stainless steel, silicone, bicarbon, polytetrafluoroethylene, tantalum, titanium, or nickel-titanium alloy. Conversely, materials may be specifically chosen to be bioabsorable so as to obviate the need for removal.

A variation of the catheter of the invention eliminates the problems of conventional intermittent catheterization by using helix or rotational technology that provides controlled insertion and flexibility to negotiate the urethra. The helix design accomplishes a pre-dilatation of the passageway at a steady rate that relaxes the sphincter and lessens or prevents spasm.

The catheter shaft has a short tapered helix on the distal 2cm (bladder end) of the shaft, and a drainage port at the distal end of the shaft. It is designed as a sterile, single-use, disposable item that can be used when the patient believes it is appropriate. The patient simply inserts the sterile catheter into the urethral opening and rotates the shaft to advance the catheter into the bladder. When the urine stops flowing the catheter is easily removed and disposed.

The threaded camera introducer system, briefly stated, involves a novel means for the introduction of sensors and other impliments into and through the full length of the colon. The fundamental structure of the introducer, in it's simplest form, is a large, soft, flexible worm-like tubular device with soft, pliant threads; the hollow core or central lumen connecting the distal and proximal ends of the tube. A camera head or other visual sensor can be arranged to "see" forward from the center of the bulbous tip on the distal end of the device. Light bundles or wires connected the camera pass through the central lumen and out the proximal end of the device to an appropriate viewing apparatus.

The distal end of the device is gently urged into the rectum. The device is rotated from outside the point of entry, to slowly advance into and through the entire length of the colon to the cecum. The helical threads pulling the device gently along the interior colon wall; the flexibility of the device allowing it to easily negotiate the major turns of the colon. The larger threads at the distal end provide the greatest grip or pull, the smaller threads closer to the proximal end contributing a lesser degree of grip or pull. The device is removed using the same method in reverse.

As illustrated in the figures, the light bundles or cables may be encased in a flexible torque tube or assembly which provides or contributes to the torsional strength necessary to rotatingly advance and withdrawn the device by turning force on the protruding portion, as close as necessary to the proximal end for full length penetration.

The interior wall of the main tubular device or introducer, may be configured to contain the torque tube or vertebra in a non-rotational manner, such that torque applied at any place on the exterior wall of the introducer is transmitted to the torque tube and hence over the full length of the device.

    1.The spiral extending the length of the device can be used to :

        a) Carry ffiuds into the colon/passage

    b) Provide vacuum to the passage way itself or vacuum within the device to facilitate the advancement of the camera or endoscope into the device.
    c) Convey light bundles or electrical wires for specific purposes
    d) Provide depth markers to assist the practitioner in determining the general position of the device within the body

2. The spiral may also be inflated with a fluid during entry to obtain full thread form and deflated to permit standard removal by pulling the device from the colon.

3. The video screen or the image on the screen may be processed to eliminate the image rotation that is produced when the screw based device is rotated into the patient.

4. The distal portion of the device should be very flexible to ensure trackability along the path of the colon/passageway.

5. The device must have sufficient torque transmission capability from the proximal to the distal end so the distal portion of the device can rotate and thus advance into the body.

6. The distal tip, zone, will have a thread height to provide the primary advancement of the device into the body while the thread height along the more proximal is to assist the entire device to advance/retract without bunching or gathering the colon wall or causing a buildup of friction as the device is advanced into the colon.

7. There may be at least three methods of containing and controlling this 160 cm long instrument to ensure it remains within the operating field :

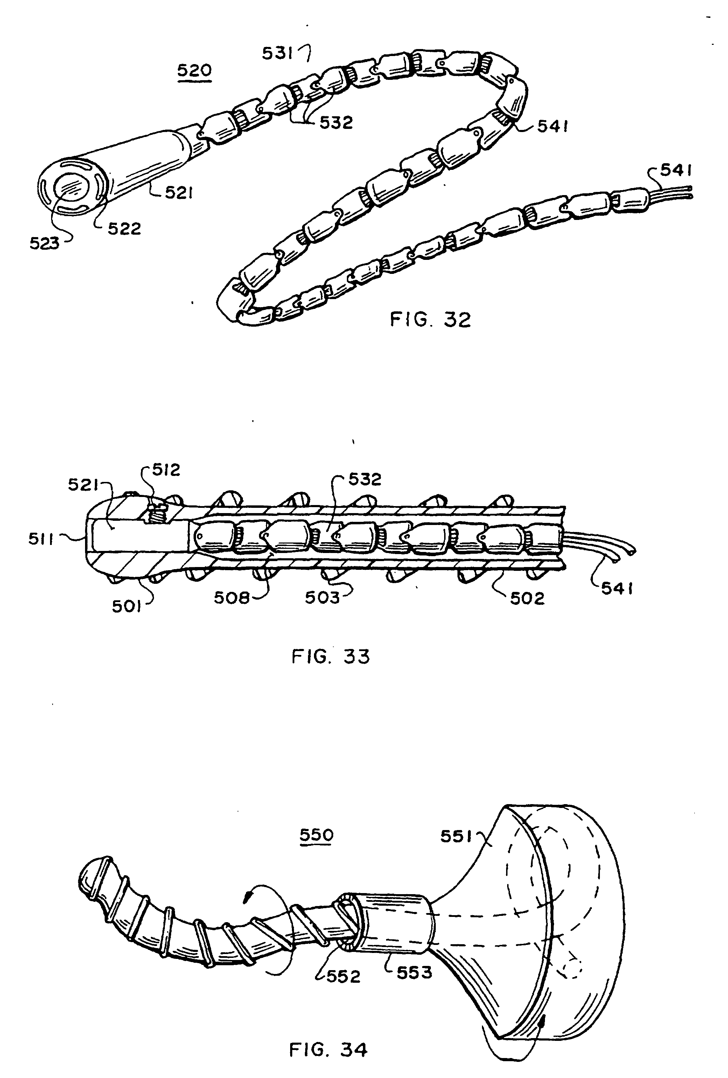

    No.1 A dispensing device as shown in Fig. 34
    No.2 A straight tubular component
    No. 3 Held by an assistant

8. Material of construction: Introducer-

-    The main body may likely be produced from polyvinylchloride plastic
-    the helix could be made of PVC which is either metal reinforced or without reinforcement
-    the window is a flat optically clear plastic made from PVC, polycarbonate, or acrylic plastic.

Camera with torque control umbilicus

- the camera body which houses both the camera and the light sources is made of stainless steel or molded with a dimensionally stable plastic such as polycarbonate
- The vertebrae which makes up the torque control umbilicus is made of a high strength thermoplastic or a metal such as stainless steel or beryllium copper.

By means of the invention, the entire colon can be examined without the need for a conventional colonoscope or endoscope, and without the attendant expertise, pain, medication, post procedure recovery time, and cost. The means and method of the invention requires less training and has far greater likelihood of reaching the cecum, (far end of the colon), than conventional tools and procedures. Other body cavities and passageways may be similarly examined.

The camera introducer catheter can be used in four different modes:

1. As an INTRODUCER

o To convey a camera assembly along the entire colon to screen patients for polyps, lesions, cancer sights and other maladies.
o Entire colon can be examined without the need for a conventional colonoscope/endoscope
o A total examination of the colon can be successfully performed with significantly less manipulation technique, pain, medication and post procedure recovery time.
o Requires less training and has greater success in reaching the cecum, ( far end of the colon).
o Is a single-use disposable device. The expensive camera with its torque controlled umbilicus can be used indefinitely.
o Procedure is less expensive when compared to the cost of cleaning and repairing the conventional endoscopes and amortizing the cost of today's costly video processing unit.
o The procedure can be successfully performed by less specialized, less expensive individuals.
o The INTRODUCER is supplied sterilized and ready for use - today's devices are almost totally used non-sterile.

2. As a more CONVENTIONAL STYLE ENDOSCOPE
By adding a screw thread to an endoscope that may have the following functions:

o Tip articulation
o Air & water delivery
o Suction of fluids
o Illumination of passages
o Imaging capability
o Drug delivery
o Accessories

3. As a HYBRID CATHETER having some of the functions and features of the more CONVENTIONAL ENDOSCOPE and/or the INTRODUCER style built into the device for procedure specific procedures. Also, could be used in conjunction with or independent of conventional endoscopic devices and accessories.

4. As a TRANSPORTOR or introducer to deliver a conventional endoscope to any location of the colon or other passageway. This can occur by:

a) Providing a fluid tight protection for the endoscope.
b) Providing a means for the endoscope to exit the INTRODUCER to perform diagnostic/Therapeutic procedures normally done with the endoscope.

Still other objects and advantages of the present invention will become readily apparent to those skilled in this art from the following detailed description, wherein I have shown and described preferred and other embodiments of the invention, simply by way of illustration of the best mode contemplated by me on carrying out my invention. As will be realized, the invention is capable of other and different embodiments, and its several details are capable of modifications in various obvious respects, all without departing from the invention.

BRIEF DESCRIPTION OF THE DRAWINGS

Fig. 1 is an illustration of the lower abdominal anatomy of a male subject, with the threaded portion of the catheter of Fig. 2 extending into the bladder.
Fig. 2 is a perspective view of a threaded catheter for a male.
Fig. 3 is a cross sectional view of the threaded portion of the catheter of Fig. 2.
Fig. 4 is a illustration of the threaded end of the catheter of Fig. 1 engaged in the urethra.
Fig. 5 is a perspective view of a threaded catheter for a female.
Fig. 6 is a cross sectional view of the threaded portion of the catheter of Fig. 5.
Fig. 7 is a perspective view of a threaded catheter and flexible shaft stylet with which it is installed.
Fig. 8 is a cross section of the tip of the catheter of Fig. 7, showing the non-rotational fitment that receives the tip of the stylet of Fig. 7.

Fig. 9 is a perspective view of the tip of the stylet of Fig. 7 that is insertable into the fitment of Fig. 8.

Fig. 10 is a diagrammatic longitudinal cross section view of a threaded balloon catheter showing the thread element inside the inflated balloon, with lumens shown as dashed lines.

Fig. 11 is a cross section view of the shaft of the catheter of Fig. 10, showing the central drain lumen and the smaller inflation lumen.

Fig. 12 is a longitudinal cross section view of the distal end of the catheter of Fig. 10, showing the balloon contracted around the helical element.

Fig. 13 is a side elevation of a threaded dilator.

Fg. 14 is a side elevation of a threaded occluder.

Fig. 15 is a side elevation of another variation of a threaded occluder.

Fig. 16 is a perspective view of a threaded stent, dashed lines showing an internal sidewall reinforcement member and a bushing with a hexagon drive socket.

Fig. 17 is a cross section view of the stent of Fig. 16.

Fig. 18 is a proximal end view of the stent of Fig. 16, with the hexagon drive socket visible at the center.

Fig. 19 is a perspective view of a stylet with a grip on it's proximal end and a hexagon drive tip on it's distal end.

Fig. 20 is a perspective view of the hexagon drive tip of the stylet of Fig. 19.

Fig. 21 is a perspective view of a stent-follower with a helical element at it's distal end.

Fig. 22 is a cross section closeup view of the distal end of the stent-follower of Fig. 21, showing the hidden portion of the bushing with it's hexagonal drive aperture in dashed lines.

Fig. 23 is a cross section view of a stent with flow control, showing the coiled wall reinforcement member acting as a spring on the ball of the check valve.

Fig. 24 is a closeup perspective view of a stylet tip for operating the check valve of the stent of Fig. 23.

Fig. 25 is a diagrammatic illustration of a suprapubic catheter emplaced through the abdomen with the distal end anchored by it's helical thread in the bladder wall.

Fig. 26 is a partial side perspective view of the helical thread of the suprapubic catheter of Fig. 25, anchored by it's helical thread in a hole in the bladder wall.

Fig. 27 is a partial front perspective of the suprapubic catheter of Figs. 25 and 26 anchored in a hole in the bladder wall; the hole being stretched and deformed to fit tightly about the tube and thread of the catheter.

Fig. 28 is a diagrammatic view of a trocar, cannula and guide wire, used to install the suprapubic catheter of Fig. 25.

Fig. 29 is a distal end view of the suprapubic catheter of Fig. 21, showing rotational orientation markers.

Fig. 30 is a front perspective diagram of a threaded camera introducer catheter advanced into the transverse colon area.

Fig. 31A is a partial front-side perspective of the distal end of the catheter of Fig. 30, showing the larger thread height of the thread in the distal area of the catheter's length.

Fig. 31B is a partial side view of the mid section of the catheter of Fig. 30, showing the reduced thread height of the thread in other than the distal area of the catheter's length.

Fig. 32 is a perspective view of a camera assembly with a video camera or visual sensor head attached to a flexible torque tube or assembly within which run electrical cables and/or light bundles.

Fig. 33 is a partial cross section view of the distal end of the preferred embodiment of FIG. 1, with the camera assembly of Fig. 2 installed as it would be used, with textual information relating to the construction and use of the device.

Fig. 34 is a rotating container and dispensing device by which the catheter of Fig. 30 may be managed and administered during it's application to a patient.

## DESCRIPTION OF THE PREFERRED EMBODIMENT

To those skilled in the art, the invention admits of many variations and appellations in apparatus and methodology.

By way of example, there is provided in accordance with the present invention, a rotate-to-advance structure and methodology applicable to a range of medical devices that have here-to-fore relied entirely or substantially on a push-to-advance mentality. Such devices include catheters, dilators, and occluders for mammalian genito-urinary or gastro-intestinal passages such as the urethra or ureter, for the usual purposes associated with such devices where no incising or rupture of passage walls or membranes is intended.

## CATHETERS:

Referring now to Figs. 1, 2 and 3, threaded catheter 101 for males is made up of tube 102 with external thread 103, attachable to flow control device 104. Tube 102 is extruded from polyurethane material, has an inside diameter of 0.06 inches, an outside diameter 103d of 0.125 inches, and is approximately 13 inches long. The durometer as measured on the smooth, outside wall of the tube is 85 Shore A. Distal end 105 is closed off, with it's tip rounded to a uniform radius of about 0.06 inches. Proximal end 106 of tube 102 is simply cut off square and attached to flow control device 104. Tube 102 is sufficiently strong such that when the majority of its length is contained within the urethra, it will withstand and transmit torque as applied by finger force at the lower end external of the urethra, to the thread.

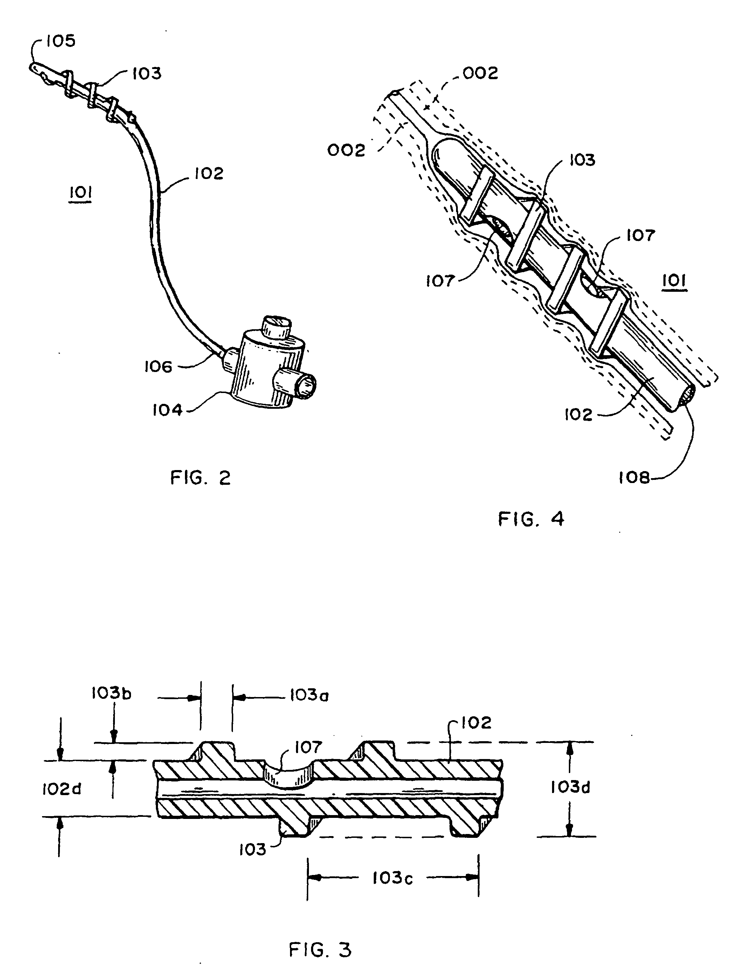

Referring to Figs. 2 and 3, external thread 103 is formed from a strip of polyurethane material with a rectangular cross section of width 103a, 0.05 inches, and height 103b, 0.032 inches, and continuously attached over it's length to tube 102 starting 0.2 inches from distal end 105 and extending four complete turns around tube 102 in a clockwise direction towards proximal end 106 at a uniform pitch 103c of 0.25 inches, resulting in a four-turn thread or helix about one inch long.

It is readily apparent from the dimensions of Figs. 2 and 3 that the thread height 103b of catheter 101 is greater than twenty percent (20%) of the 103d thread diameter. This relative height is necessary to expand and penetrate the longitudial folds of the urethra to a sufficient depth to achieve a useful grip by the thread.

The diameter of the helix formed by thread 103 of catheter 101 is referred to as thread diameter 103d, and is equal to two thread heights 103b plus the outside diameter 102d of catheter tube 102, or in this case 2 times .032 inches pius 125 inches or approximately 0.19 inches. The circumference C of the helix formed by thread 30 is calculated as $\Pi$ (pi) times thread diameter 103d, or in this case 3.14 times 19 or approximately 0.6 inches.

$$C = \Pi \cdot 103d$$

The ratio R of thread pitch 103c, 0.25 inches, to the circumference of thread diameter 103d, at 0.6 inches, is much less than 1 to 1, thereby improving the leverage of the screw thread for converting rotation into longitudinal pulling power as compared to larger ratios.

$$R = 103c \div C$$

The shoulders of thread 103 have a radius of 0.015 inches. In small quantities, thread 103 may be attached to tube 102 by wicking tetrahydrofuran (THF) solvent under the thread using a fine hollow tube. Catheter 101 may be molded in large quantities with thread 103 being an integral part of the molded structure.

Referring to Fig. 4, two drainage ports 107, connecting to lumen 108, are oval in shape, the major axis of the oval being parallel with the axis of tube 102 and about 1.5 times the minor axis, which is about equal to the diameter of the lumen. The two ports are configured 180 degrees apart radially, and spaced longitudinally to fit between the turns of thread 103.

Both ends of thread 103 are tapered from zero to full height in one-half turn of the helix, to facilitate gentle, gradual displacement of urethra wall 002 by thread 103 when catheter 101 is rotated clockwise for advancement into the urethra and counterclockwise for retraction. The difference between thread width 103b and pitch 103c shown in Fig. 3 is sufficient that the urethra wall 002 does not bridge between adjacent turns of thread 103, but rather is only displaced in a manner closely conforming to the cross section of thread 103, thereby providing the longitudinal grip on urethra wall 002 for advancing and retracting the catheter.

Referring to Fig. 1, catheter 101 is shown in proper position for draining bladder 004, after it has been advanced through the urethra 006 until the helix passes out of the urethra into the bladder.

It is apparent from the anatomy shown in Fig. 1, that thread 103 must be limited in length to be advanced to any point above the sphincter 008, so that the sphincter may contract directly onto the smooth, round, exterior of tube 102, thereby preventing leakage around the tube, and further constraining catheter 101 from migrating or being forced out of the urethra by pressure from urine in the bladder. It is further apparent from the figure that there is a limit to the length of thread 103 on a catheter that can be advanced to a position above the sphincter 008, not more than about six turns within the optimal range of thread pitch, and still fit within the bladder 004 without interference. A limited length of thread 103 also localizes the area of pulling force to the upper end of catheter 101, assuring that the trailing length of the catheter is drawn, not pushed, through the passage.

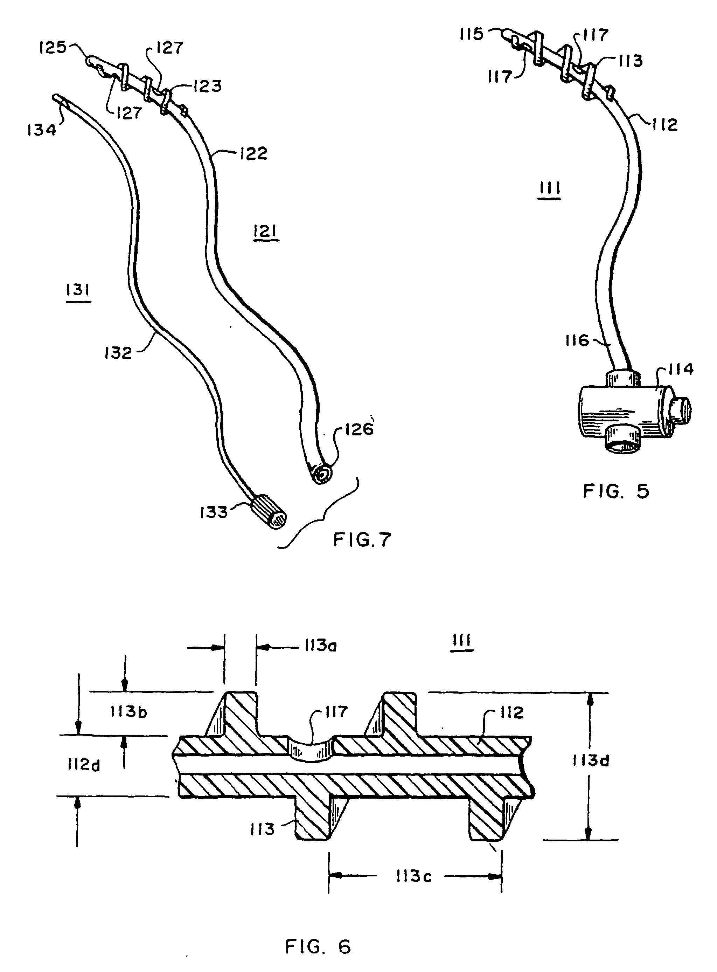

Referring to Figs. 5 and 6, threaded catheter 111 for females, similar to catheter 101 for males, is made up of tube 112 with thread 113, attachable to flow control device 114, Tube 112 is extruded from polyurethane material, has an inside diameter of 0.063 inches, an outside diameter 112d of 0.125 inches, and is approximately seven inches long. The durometer as measured on the smooth, outside wall of the tube is 85 shore a. Distal end 115 is closed off, it's tip rounded to a uniform radius of about 0.06 inches. Proximal end 116 of tube 112 is simply cut off square and attached to flow control device 114. Tube 112 is sufficiently strong such that when the majority of its length is contained within the urethra, it will withstand and transmit torque as applied by finger force at the lower end external of the urethra, to the thread or helix.

Referring to Figs. 5 and 6, thread 113 of catheter 111 is formed from a strip of polyurethane material with a rectangular cross section of width 113a of 0.05 inches and height 31b of 0.10 inches, attached to tube 112 starting 0.2 inches from distal end 115 and extending four turns around tube 112 in a clockwise direction towards proximal end 116 at a uniform pitch 113c of 0.25 inches, resulting in a four-turn thread or helix about one inch long.

It is readily apparent from Figs. 5 and 6 that the thread height 113b of catheter 111 at 0.10 inches, is much greater than twenty percent (20%) of tube diameter 112d, at 0.125 inches. This relative thread height is necessary in order to expand and penetrate the longitudial folds of the female urethra sufficiently far to achieve a useful grip by the thread.

Similar to the description of threaded catheter 101, the diameter 113d of the helix formed by thread 113 is equal to two thread heights 113b plus the diameter 112d, or in this case 2 times 10 plus 125 or approximately 0.33 inches. The circumference C of the helix formed by thread 113 is calculated as Π (pi) times the thread diameter 113d, or in this case 3.14 times 0.33 or approximately 1.0 inches. The ratio R of thread pitch 113c, at 0.25 inches, to the circumference C, at 1.0 inches, is again much less than 1 to 1, thereby improving the leverage of the thread for converting rotation into longitudinal pulling power as compared to larger ratios.

The shoulders of thread 113 have a radius of 0.015 inches. Catheter 111 may be constructed or fabricated by the same means as catheter 101.

Referring to Fig. 5, two side drainage ports 117, connecting to lumen 118, are oval in shape, the major axis of the oval being parallel with the axis of tube 112 and about 1.5 times the minor axis, which is about equal to the diameter of the lumen. The two sideports 117 are configured 180 degrees apart radially, and spaced longitudinally to fit between the turns of the thread.

Referring to Fig. 5 and 6, the ends of thread 113 are tapered from zero to full height in three-quarters turn of the helix, to facilitate gentle, gradual displacement of urethra wall by the thread when catheter is rotated clockwise for advancement and counterclockwise for retraction. The difference between width 113a and pitch 113c is sufficient that the urethra wall does not bridge between adjacent turns, but rather is displaced in a manner closely conforming to the profile of the thread, thereby providing the longitudinal grip on the urethra wall for advancing and retracting the catheter, the same as catheter 101 of Figs. 2 and 3.

The optimal position for threaded catheter 111 for draining the bladder of a female subject is where it is advanced through the urethra until the thread passes out of the urethra into the bladder, similar to how catheter 101 is illustrated in Fig. 1, but for females.

A detailed method of self-administration of the urethra with the appropriate respective threaded catheter 101 or 111, or other similar threaded devices, is explained:

> The user assembles materials including a sterile threaded catheter 101 or 111, a container for urine, soap and water, if the catheter is not pre-lubricated then a water soluble lubricant, a mirror (for females), and tissues. The user will then wash the hands and urethral opening with soap and water, squeeze out a small amount of lubricant into clean tissue, dip the distal end tip of the catheter into the lubricant, and manually engage the tip of the catheter into the urethral opening, (the mirror may be helpful for females to assist in locating the opening).

The user will then gently push and turn the catheter in far enough to engage the thread about one full turn with the urethra, and gently rotate the tube of the catheter in the direction of the thread, preferably clockwise, to advance the catheter into the urethra until urine appears in the tube. The user then pauses to drain the bladder, directing the urine into the container, then resumes rotation of the catheter until it is no longer advanced by the rotation, indicating that the thread of the catheter has passed into the bladder and the catheter is in proper position.

The user then places a flow control device on the proximal end of the catheter and empties the bladder periodically as required. The catheter is removed when appropriate using similar precautions for cleanliness and containment, by rotating the catheter in a direction opposite the direction of insertion, presumably counterclockwise.

Referring to Figs. 7, 8 and 9, another embodiment contemplated by the claims is illustrated by catheter 121, which is made up of tube 122 with thread 123 applied in the form of a helix, and utilizing flexible shaft stylet 131 as an insertion and retraction tool. Stylet 131 has grip 133 at the proximal end, for turning. Tube 122 is configured with non-rotational fitment 124 near distal end 125 so that stylet 131 can be inserted through proximal end 126, up through lumen 128 of tube 122, and tip 134 of stylet 131 be then engaged with fitment 124 in a manner that allows rotation of grip 133 in one direction to rotate catheter 121 for advancement into the urethra, and in the other direction for retraction.

The flexible shaft 132 of stylet 131 is sufficiently strong such that when it is fully inserted into catheter 121, it will withstand and transmit torque as applied by finger force at the proximal end knurled knob grip 133 external of the urethra, to the thread 123. Stylet 131 is removed after catheter 121 is installed, and reinserted for retracting the catheter when required.

Fitment 124 is an elongated collar with a multifaceted interior wall, securely anchored within tube 122, and configured to receive in a non-rotational relationship tip 134. Tip 134 is configured with a corresponding elongated, multi-faceted exterior shape and rounded end, to readily enter fitment 124.

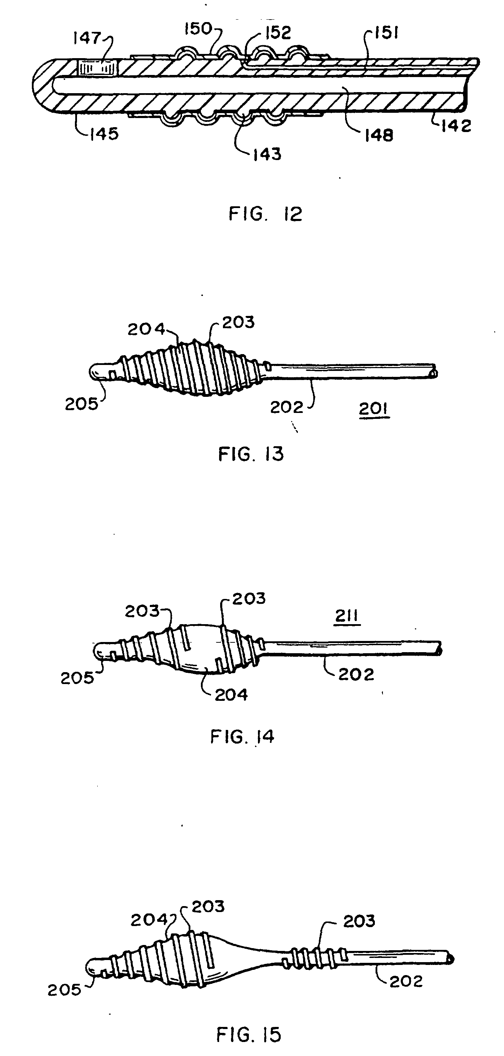

Referring to Fig. 10, 11 and 12, a threaded foley-type catheter 141 of the invention is made from polyurethane material. It has a flexible tube 142 with an axial drainage lumen 148 running from a drainage port 149 to it's proximal end 146a, and a thread 143 applied to it's external surface near it's distal end 145 in the manner of threaded catheters previously described. Catheter 141 has a thin-walled inflatable elastic balloon 150 encasing the helical thread 143 and sealed to tube 142 above and below the thread 143. Drainage port 149 is located above or distally from balloon 150. A smaller inflation lumen 151 within tube 142 communicates between inflation port 152 within the envelope of balloon 150 to the distal end 146b of the catheter. Lumens 148 and 151 are isolated from each other, as indicated by Figs. 11 and 12.

Balloon 150, when uninflated, is normally contracted tightly about helical element 143 as illustrated in Fig. 12, and may be inflated as in Fig. 10 by injecting fluid through lumen 151 into the balloon cavity 153. The flexible tube 142 is of sufficient torsional strength to withstand and transmit rotational finger force at the proximal end to thread 143.

DILATORS AND OCCLUDERS:

Referring now to Figs. 13, 14 and 15, dilator 201 and occluders 211 and 221 are similarly constructed by configuring the upper end 205 of a flexible shaft 202 with tapered bulb 204 near it's distal end, and disposing thereon one or two sections of thread 203. These threads are similar to thread 103 on catheter 101 of Figs. 2 and 3, wherein the height of the thread is at least twenty percent (20%) of the diameter of the shaft 202, and the ratio of thread pitch to the circumference of the thread diameter at any given point on the bulb or shaft is less than one to one (1/1). The ends of threads 203 are tapered for ease of advancing and retracting, again similar to the threaded catheter of Figs. 2 and 3.

Dilator 201, of Fig. 13, is configured with multiple turns of thread 203 extending over both ends of tapered bulb 204, and is used to dilate a constricted passage by being rotatingly advanced and retracted through the obstructed area of the passage in the same fashion as the threaded catheters of the invention.

Occluder 211, of Fig. 14, is configured with two sections of thread 203, leaving the midsection or bulbous portion of tapered bulb 204 smooth and round in order to provide a uniform occluding surface. This occluder is used to plug or constrict a passageway at an interior point; being rotatingly advanced to and retracted from that point in the same fashion as the threaded catheters of the invention.

Occluder 221, of Fig. 15, is configured with two sections of thread 203, the lower or proximal end thread 203 being disposed on the shaft 202 below the tapered bulb 204, leaving the lower tapered end of bulb 204 smooth and round in order to provide a uniform occluding surface. This occluder is used to plug a passageway at the interior end neck or entrance; being rotatingly advanced until the tapered bulb passes entirely through the passage while the lower thread remains engaged in the passage, and being then rotatingly retracted to seat the tapered bulb against the neck of the passage. The occluder is then rotatingly retracted when appropriate.

STENTS:

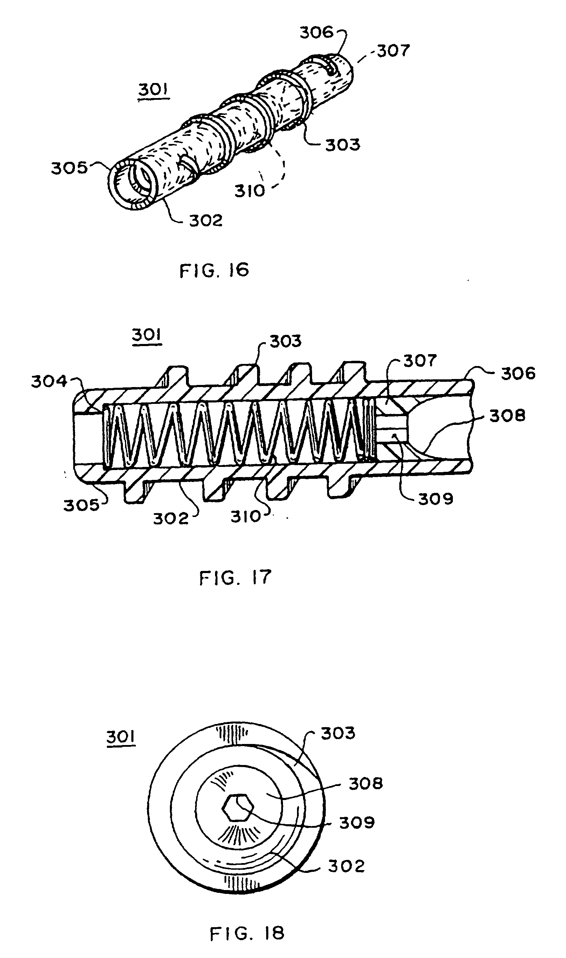

Referring now to Figs. 16-18, a threaded urethral stent 301 made from polyurethane material has a tube 302 with an external thread 303 of uniform pitch. Thread 303 is similar to thread 103 on catheter 101 of Figs. 2 and 3, wherein the height of the thread is at least twenty percent (20%) of the diameter of the shaft 202, and the ratio of thread pitch to the circumference of the thread diameter is less than one to one (1/1). The ends of thread 303 are tapered for ease of advancing and retracting through a passage. There is an interior shoulder 304 at the distal end 305 of the stent, and a bushing 307 of relatively harder material with a tapered interior wall 308 extending from the bushing's full diameter at one end to a uniform hexagonal aperture 309, bushing 307 being affixed within the proximal end 306 of the stent and oriented with tapered wall 308 extending proximally from aperture 309. Coiled sidewall reinforcement member 310 is secured within the remaining length of stent 301 by bushing 307 and interior shoulder 304. Alternative embodiments may have a midsection of the thread being tapered to a lesser height or no height, to provide a "waist" for gripping by a muscular zone such as the prostate or sphincter. Also, reinforcement member 310 could be configured or molded into the sidewall tube 302.

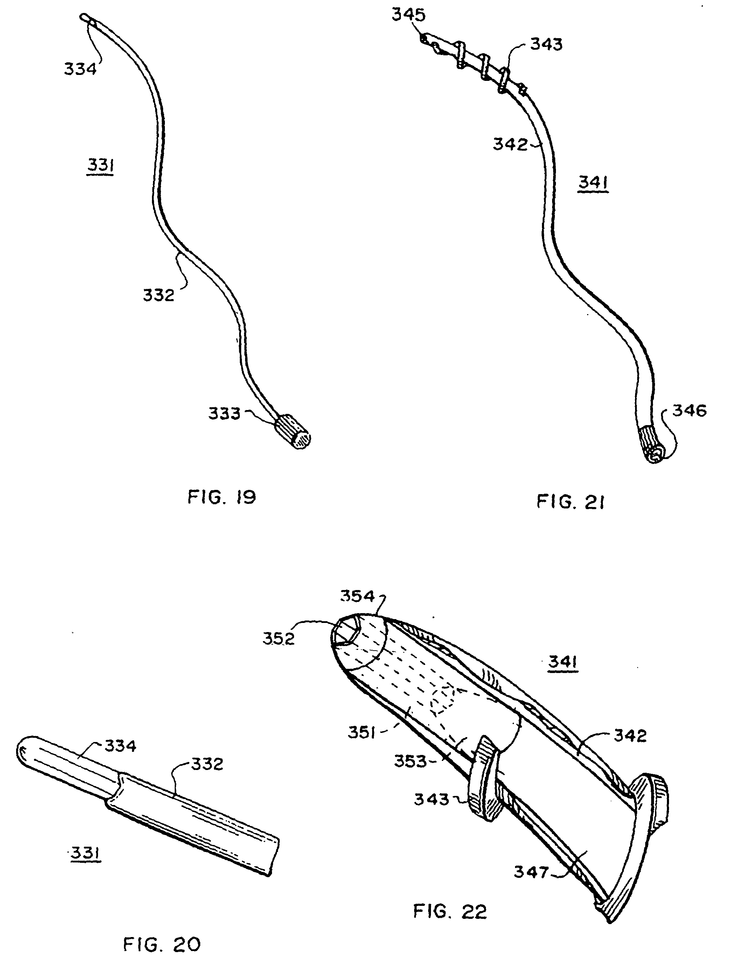

Referring now to Figs. 19 and 20, stylet 331, similar to stylet 131 of Fig. 7, has flexible shaft 332 with grip 333 at the proximal end for turning, and hardened hexagon tip 334 at the distal end which closely fits into aperture 309 of stent 301 in a non-rotational manner for emplacement of the stent by the method of the invention. The flexible shaft 332 of the stylet is sufficiently strong such that when tip 334 is inserted into aperture 309, the shaft will withstand and transmit torque as applied by rotational finger force at grip 333 to thread 303.

Referring now to Fig. 21 and 22, threaded stent-follower 341 has a flexible tube 342, lumen 347 of which is sized to accept the ready insertion of tip 334 and shaft 332 of stylet 331 of Fig. 19. Tube 342 is of sufficient torsional strength to accept and transmit rotational finger force at it's proximal end 346 to it's distal end 345. A thread 343 of uniform pitch and not more than six turns is applied to the external surface of tube 342 near distal end 345. Thread 343 conforms to the same twenty percent (20%) rule of thread height to tube diameter, and ratio of thread pitch to thread circumference of less than one to one (1/1), as thread 103 in Figs. 2 and 3 as described above. The ends of thread 343 are tapered for ease of advancing and retracting.

Referring to Fig. 17 and Fig. 22, bushing 351 has a uniform hexagonal aperture 352 the same size as aperture 309 in bushing 307 of stent 301, and a tapered interior wall 353 extended from it's full diameter at it's proximal end to aperture 352. Bushing 351 also has an external tapered tip 354 at it's distal end. Bushing 351 is affixed within the distal end 345 of tube 342 with tip 354 protruding, such that the distal end 345 of stent-foflower 341 mates with a self-centering action with the proximal end of stent 301 when the two devices are brought into contact with approximate axial alignment. When stent-follower 341 and stent 301 are thus mated, tip 334 of stylet 331 may be extended through aperture 352 and into aperture 309, thereby locking stent 301 and stent-follower 341 into a fixed rotational relationship. In this condition, the rotation of the proximal end of stylet 331 and stent-follower 341, causes the concurrent rotation of stent 301, whether to rotatingly advance or retract the stent. Stylet 331 may be withdrawn and stent-follower 341 rotatingly retracted, leaving stent 301 positioned at any useful point within a passageway.

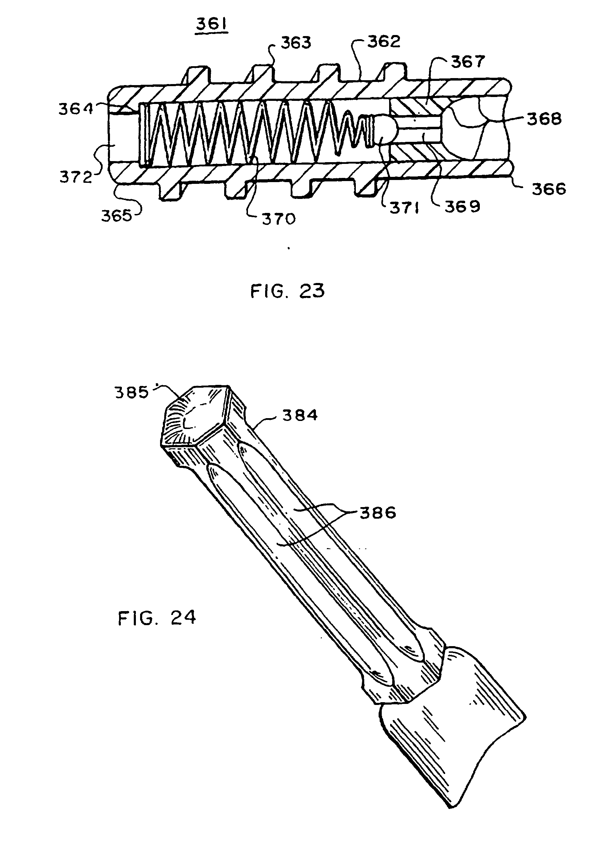

Referring now to Fig. 23, threaded stent 361, shown in cross section, incorporates means for flow control. The stent has a tube 362 made from a section of extruded polyurethane tubing material, with thread 363 of uniform pitch and not more than six turns applied to it's external surface. Thread 363 conforms to the same twenty percent (20%) rule of thread height to tube diameter, and ratio of thread pitch to thread circumference of less than one to one (1/1), as thread 103 in Figs. 2 and 3 as described above.

There is an interior shoulder 364 at the distal end 365 of stent 361, a bushing 367 of relatively harder material with a tapered interior wall 368 extending from the bushing's full diameter at one end to a uniform hexagonal aperture 369, bushing 367 being affixed within the distal end 366 of stent 361 and oriented with tapered wall 368 extending proximally.

A coiled sidewall reinforcement member 370 and a check ball 371 are secured within the remaining length of stent 361 by bushing 367 and interior shoulder 364 so that coiled member 370 holds ball 371 in compression against the upper end of bushing 367 in the manner of a check valve, which prevents outward flow through the lumen 372 of the stent. Coiled member 370 may be compressed by upward movement of ball 371, thereby opening the check valve to flow.

Referring to Figs. 19, 21, 23 and 24, alternate hexagonal tip 384 for stylet 331 has a slightly concave proximal end 385 and flutes 386. When used in conjunction with stent-follower 341 to actuate the check valve of stent 361, tip 384 is be inserted through aperture 369 of stent 361 to push ball 371 upward against coil member 370, thereby opening the check valve function and permitting outward flow of fluid through flutes 386 and aperture 369 into and through stent-follower 341 .

SUPRAPUBIC:

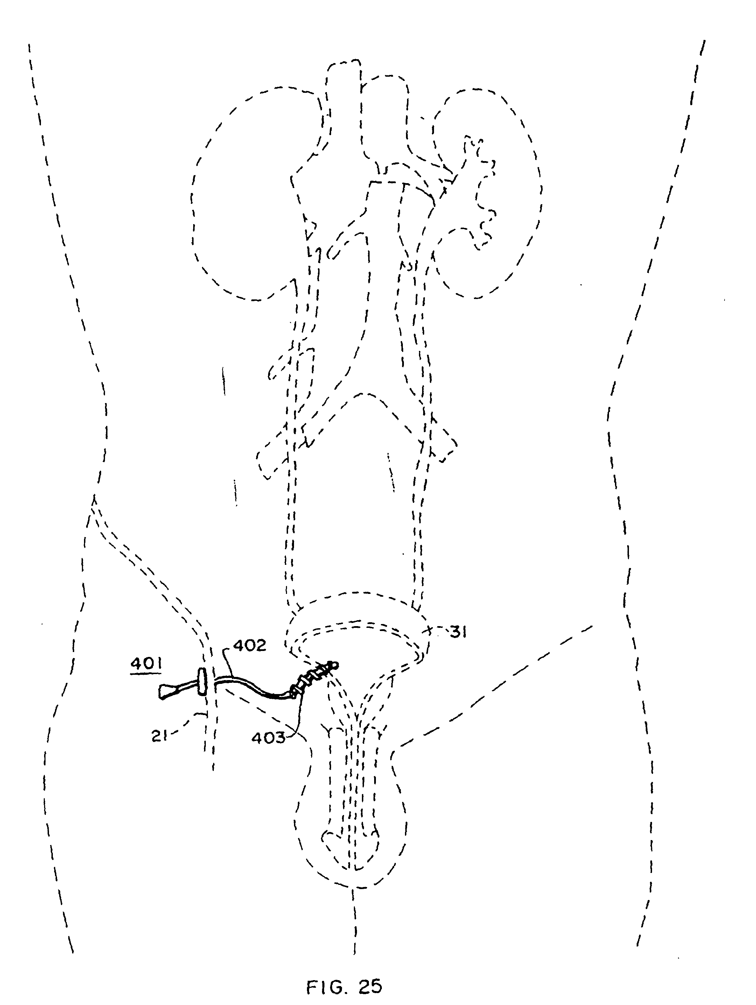

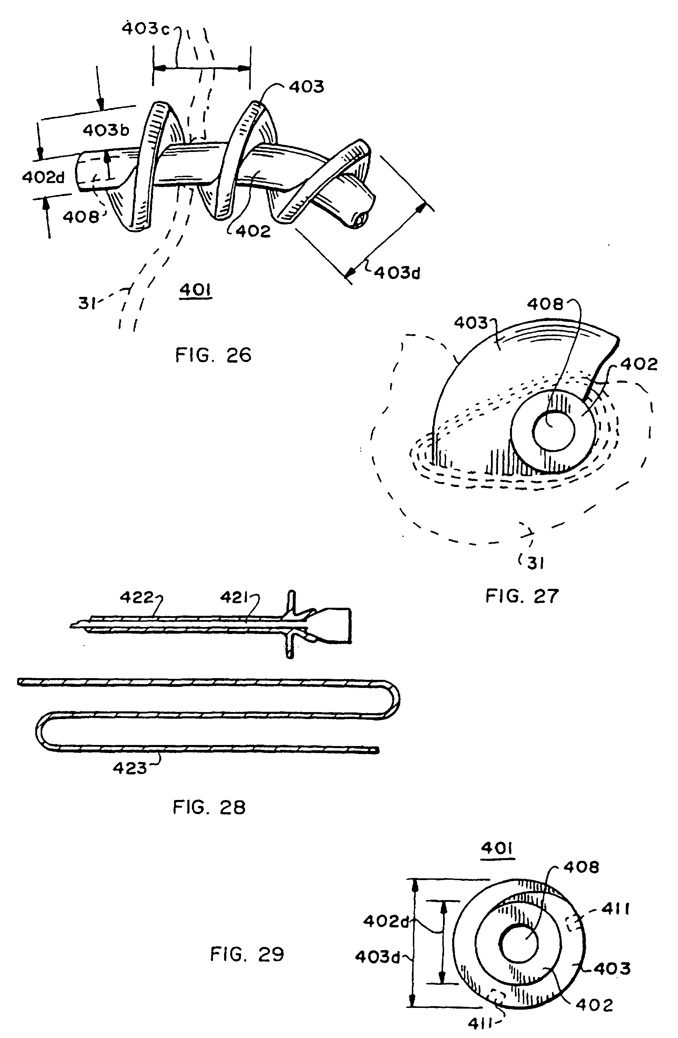

Referring now to Figs. and 25 -29, the threaded suprapubic catheter 401 of fig. 25 and 26 is constructed with a flexible tube 402 with a lumen 408 connecting axial ports at the proximal end and the distal end, and an external thread 403 of uniform pitch applied at it's distal end. As described for catheter 101 of Fig. 2 and 3, the ratio of thread pitch 403c to the circumference of thread diameter 403d is much less than one to one (1/1). Tube 402 is of sufficient torsional strength to accept and transmit rotational finger force applied at the proximal end to the distal end. The ends of 403 are tapered for ease of advancing and retracting the catheter through the abdomen and into the bladder wall.

Referring to Figs. 26 and 27, relative thread height 403b, as a percentage of tube diameter 402d, is necessarily much greater than in the case of catheter 101 of Figs. 2 and 3; greater than fifty percent (50%). The suprapubic catheter is being advanced by the rotation of thread 403 along an unlined path through the abdomen, and being anchored against longitudinal displacement by the low pitch 403c of thread 403 as to it's circumference in a hole pierced into organ wall 031 that must encompass tube 402 plus thread 403 passing through the plane of the organ wall 031. This is distinguished from the longer gripping surface available in a lined passage way as is the case for catheter 101 of Fig. 4.

Referring to to Fig. 28, a method by which suprapubic catheter 401 is applied is conventional to the extent that trocar 421 and cannula 422 are used with ultrasound or fluoroscopy to create the path through abdomen wall 021 into the bladder organ 031, trocar 421 is removed and temporary guide wire 423 is then inserted through cannula 422, extending from outside the abdomen wall 021 to inside the bladder organ 031. Cannula 422 is then withdrawn, leaving guidewire 423 as a connecting path extending from outside the body, passing through the abdominal wall 021, and into the bladder organ 031.

Suprapubic catheter 401 is then threaded through it's axial ports onto the proximal end of guide wire 423, and gently started into the abdomen wall 021 with a rotating motion about one turn until thread 403 is firmly engaged. The catheter is then rotatingly advanced along the guide wire through the unlined pathway in the same manner as other threaded devices of the invention, until thread 403 penetrates the wall of organ 031 about one full turn, as determined by ultrasound, fluoroscopy or equivalent means. The distal end of catheter 401 is secured in a non-rotatable fashion to abdomen wall 021 using conventional adhesive means or equivalent means, thereby locking thread 403 at the distal end of the catheter in position in the wall of organ 031. Guide wire 423 is withdrawn. Threaded suprapubic catheter 401 is then available for use.

Referring to Fig. 29, radiopaque markers 411 embedded at select points displaced along the perimeter of thread 403 provide the capability for external detection and monitoring through fluoroscopy or other means of orientation and movement of the distal end of the catheter.

CAMERA INTRODUCER:

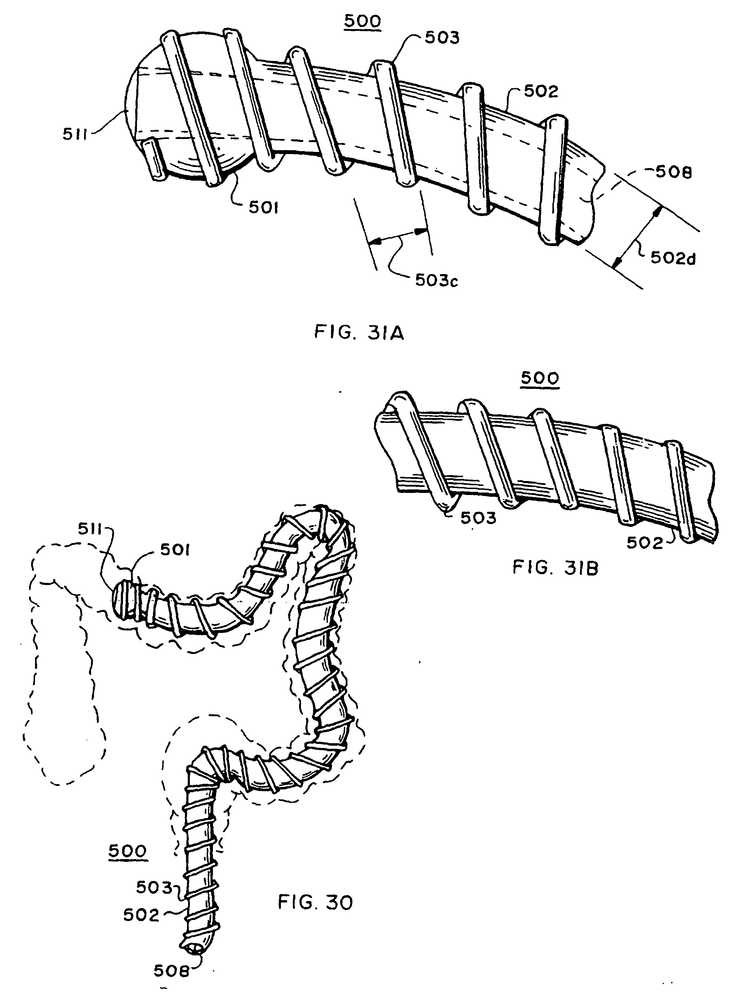

Referring to Figs. 30 and 31A, threaded camera introducer catheter 500 consists of a bulbous tip 501 connecting to a soft, flexible tube 502 which is about 5 feet long with a tube diameter 502d of one (1) inch. Lumen 508 extends from the interior face of a window 511 on the distal end of tip 501, through tip 501 and tube 502 to the proximal end of tube 502.

Referring to Fig. 31 A, external thread 503 with uniform pitch 503c of 1 inch begins at the edge of window 511, tapering to a height of about 0.16 inches, extending around tip 501 and tapering there to about 0.32 inches and continuing proximally for about 6 inches along tube 502.

Referring to Fig. 31B, thread height then tapers from a thread height of 0.32 inches to 0.16 inches and continues to the proximal end of tube 502.

Referring to Fig. 32, a camera assembly 520 consists of camera 521 with light lens 522 and image lens 523, attached to a flexible, hollow, jointed spine 531. A cable harness 541 connected to camera 521, passes through spine 531, extending out the proximal end and connecting to the necessary power, control and display equipment. Spine 531 is constructed of a chain of vertebrae 532, connected by universal joints which combine flexibility with torsional strength.

Referring to Fig. 33, camera assembly 520 is shown installed in camera introducer catheter 501, with camera 521 secured within tip 501 by set screw 512, so that the camera views forward through the window. The camera assembly and catheter are combined here as a camera introducer system..

Referring to Fig. 34, rotating container and dispensing system 550 consists of drum 551 with axial opening 552 around which handle 553 is rotatably attached. Catheter 501 is rotatingly dispensed during application by holding handle 553 and rotating drum 551 while catheter 401 is being rotatingly advanced in the subject colon.

As will be realized, the invention is capable of other and different embodiments, and its several details are capable of modifications in various obvious respects, all without departing from the invention. The objects and advantages of the invention may be further realized and attained by means of the instrumentalities and combinations particularly pointed out in the appended claims. Accordingly, the drawings and description are to be regarded as illustrative in nature, and not as restrictive.

## Claims

1. A threaded catheter (101, 111, 121, 141) for catheterizing mammalian genito-urinary and gastro-intestinal passages, said catheter comprising a flexible catheter tube (102, 112, 122, 142) having a lumen (108, 128, 148) extending from a drainage port (107, 117,127, 147) at the distal end (105, 115, 125, 145) to the proximal end (106, 116, 126, 146), said distal end of said tube configured with an external screw thread (103, 113, 123, 143), of uniform pitch, said tube having sufficient torsional strength to transmit rotational force applied at said proximal end of said tube to said thread, **characterized in that** said thread has a thread height of at least one fifth (1/5) of the outside diameter of said catheter tube and a thread pitch less than the circumference of the helix formed by said thread.

2. The threaded catheter of claim 1, said thread (103, 113, 123, 143) being no longer than six turns.

3. The threaded catheter of claim 1 said catheter tube (102, 112, 122, 142) configured to accept insertion of a flexible shaft stylet (131) through said proximal end (106, 116, 126, 146) of said tube into said lumen, the distal tip (134) of said stylet engagable in a non-rotational fitment secured in said lumen (128) of said catheter near said distal end, said stylet having sufficient torsional strength to transmit rotational force applied at the proximal end of said stylet to said tip of said stylet.

4. The threaded catheter of claim 1, said catheter (141) further comprising a thin-walled inflatable balloon (150) encompassing and terminating on said tube (142) at one end distally of said thread (143) and proximally of said drainage port (147) and at the other end proximally of said thread, an inflation lumen (151) extending from an inflation port (152) in said tube within the envelope of said balloon to said proximal end of said tube.

5. A threaded dilator (201) for dilating mammalian genito-urinary and gastro-intestinal passages, said dilator comprising a flexible shaft (202) incorporating a tapered bulb (204) near its distal end with a helical thread (203) of uniform pitch disposed thereon, said shaft (202) having sufficient torsional strength to transmit rotational force applied at said proximal end of said shaft to said thread, **characterized in that** said thread has a thread height of at least one fifth (1/5) of the outside diameter of said shaft and a thread pitch less than the circumference of the helix formed by said thread.

6. A threaded stent (301, 361) for supporting mammalian genito-urinary and gastro-intestinal passages, said stent (301, 361) comprising a flexible tube (302, 362) with an external helical thread (303, 363) of uniform pitch disposed thereon, said tube (302, 362) further comprising means for axial engagement in a non-rotational relationship with the distal end of the stylet (331), **characterized in that** said thread has a thread height of at least one fifth (1/5) of the outside diameter of said tube and a thread pitch less than the circumference of the helix formed by said thread.

7. The threaded stent of claim 6, said stent (361) further comprising means for flow control, said means being a check valve (371) incorporated into said stent, said check valve (371) being actuable by use of a stylet (331).

8. A threaded suprapubic catheter (401) for drainage of a genito-urinary organ, said catheter comprising a flexible catheter tube (402) having a first axial port at its distal end and a second axial port at its proximal end and a lumen (408) communicating there between, said distal end of said tube configured with an external screw thread (403) of uniform pitch, said tube having sufficient torsional strength to transmit rotational force applied at said proximal end of said tube to said thread, **characterized in that** said thread has a thread height of at least one half (1/2) of the outside diameter of said catheter tube and a thread pitch less than the circumference of the helix formed by said thread.

9. The threaded suprapubic catheter of claim 8, said thread (403) being no longer than six turns in length.

10. The threaded suprapubic catheter of claim 8, said catheter having externally detectable means for determining orientation of said distal end.

11. A threaded cathether of any of claims 1 to 4 and 8 to 10 wherein the opposite ends of said thread are tapered from a threaded height of at least one fifth (1/5) of the outside diameter of said tube to zero height to facilitate advancement and retraction of the catheter in a mammalian genito-urinary or gastro-intestinal passage.

12. A threaded camera introducer system for accessing the gastro-intestinal tract, comprising a flexible catheter tube (502) with a lumen (508), and an external thread (503) of uniform pitch over the length of said tube, **characterized in that** the system further comprises a bulbous tip (501) at the distal end of said tube, said lumen (508) extending from the interior face of a window (511) on the distal end of said bulbous tip to the proximal end of said tube and **in that** said external thread (503) also extends along said tip and has a thread height of at least one fifth (1/5) of the outside diameter of said tube along a portion of the length of said tube and a thread height of less than one fifth (1/5) of the outside diameter of said tube along the length of said tip.

13. The threaded camera introducer system of claim 12, said system further comprising a camera (520) attached to the distal end of a flexible hollow spine (531), a cable harness (541) extending from said camera (521) through said spine (531) and out the proximal end thereof, said camera (521) and said spine (531) being insertable and securable within said tube (502) with said camera (521) configured to view through said window (511), said spine (531) having sufficient torsional strength to transmit rotational force applied at said proximal end of said spine to said distal end.

14. The threaded camera introducer system of claim 12, further comprising a drum (551) with an axial opening (552) around which a handle (553) is rotatably attached, said drum (551) sized to hold said camera introducer system in a coiled configuration, said drum rotatingly dispensing said system through said axial opening while said handle is held in a non-rotational grip.

**Patentansprüche**

1. Gewindekatheter (101, 111, 121, 141) zur Katheterisierung von urogenitalen und gastrointestinalen Passagen des Säugers, wobei der Katheter eine flexible Katheterröhre (102, 112, 122, 142) mit einem Lumen (108, 128, 148) umfasst, welches sich von einer Drainageöffnung (107, 117, 127, 147) am distalen Ende (105, 115, 125, 145) zum proximalen Ende (106, 116, 126, 146) erstreckt, wobei das distale Ende der Röhre mit einem externen Schraubgewinde (103, 113, 123, 143) konfiguriert ist, mit einer gleichmäßigen Steigung, wobei die Röhre eine ausreichende Drehfestigkeit aufweist, um an dem proximalen Ende der Röhre aufgebrachte Drehkraft zu dem Gewinde zu übertragen,
**dadurch gekennzeichnet,**
**dass** das Gewinde eine Gewindehöhe von zumindest einem Fünftel (1/5) des äußeren Durchmessers der Katheterröhre aufweist und eine Gewindesteigung kleiner als der Umfang der Schraubenlinie, die von dem Gewinde gebildet wird.

2. Gewindekatheter nach Anspruch 1,
**dadurch gekennzeichnet,**
**dass** das Gewinde (103, 113, 123, 143) nicht länger als sechs Umdrehungen ist.

3. Gewindekatheter nach Anspruch 1,
**dadurch gekennzeichnet,**
**dass** die Katheterröhre (102, 112, 122, 142) so gestaltet ist, dass die Einführung eines flexiblen Wellenstiletts (131) durch das proximale Ende (106, 116, 126, 146) der Röhre in das Lumen zugelassen wird, dass die in einen nicht drehbaren Passungseinsatz eingreifende distale Spitze (134) des Stiletts in dem Lumen (128) des nahe dem distalen Ende befindlichen Katheters gesichert wird, dass das Stilett eine ausreichende Drehfestigkeit aufweist, um an dem proximalen Ende des Stiletts aufgebrachte Drehkraft zu der Spitze des Stiletts zu übertragen.

**4.** Drehkatheter nach Anspruch 1,
**dadurch gekennzeichnet,**
**dass** der Katheter (141) des Weiteren einen dünnwandigen aufblasbaren Ballon (150) umfasst, der die Röhre (142) an einem Ende distal von dem Gewinde (143) und proximal von der Drainageöffnung (147) umgibt und an dieser endet und an dem anderen Ende proximal des Gewindes ein sich von einer Aufblasöffnung (152) in der Röhre innerhalb der Umhüllung des Ballons zu dem proximalen Ende der Röhre erstreckendes Aufblaslumen (151).

**5.** Gewindedilator (201) zur Dehnung von urogenitalen und gastrointestinalen Passagen des Säugers, wobei der Dilator einen flexiblen Schaft umfasst (202), der nahe seinem distalen Ende einen konisch zulaufenden Bulbus (204) aufnimmt, mit einem darauf angeordneten schraubenförmigen Gewinde (203) mit gleichmäßiger Steigung, wobei der Schaft (202) eine ausreichende Drehfestigkeit aufweist, um an dem proximalen Ende des Schaftes aufgebrachte Drehkraft zu dem Gewinde zu übertragen,
**dadurch gekennzeichnet,**
**dass** das Gewinde eine Gewindehöhe von zumindest einem Fünftel (1/5) des äußeren Durchmessers des Schaftes aufweist und eine Gewindesteigung kleiner als der Umfang der von dem Gewinde gebildeten Schraubenlinie.

**6.** Gewindestent (301, 361) zur Stützung von urogenitalen und gastrointestinalen Passagen des Säugers, wobei der Stent (301, 361) eine flexible Röhre (302, 362) umfasst, auf der ein externes spiralförmiges Gewinde (303, 363) mit gleichmäßiger Steigung angeordnet ist, wobei die Röhre (302, 363) des Weiteren Mittel zum axialen Eingriff in einer nicht drehbaren Beziehung mit dem distalen Ende des Stiletts (331) umfasst,
**dadurch gekennzeichnet,**
**dass** das Gewinde eine Gewindehöhe von zumindest einem Fünftel (1/5) des äußeren Durchmessers der Röhre aufweist und eine Gewindesteigung kleiner als der Umfang der von dem Gewinde geformten Schraubenlinie.

**7.** Gewindestent nach Anspruch 6,
**dadurch gekennzeichnet,**
**dass** der Stent (361) des Weiteren Mittel zur Durchflusssteuerung umfasst, wobei das Mittel ein in dem Stent enthaltenes Rückschlagventil (371) ist, wobei das Rückschlagventil (371) durch Verwendung eines Stiletts (331) bedienbar ist.

**8.** Suprapubischer Gewindekatheter (401) zur Drainage eines urogenitalen Organs, wobei der Katheter eine flexible Katheterröhre (402) umfasst, mit einer ersten axialen Öffnung an deren distalen Ende und einer zweiten axialen Öffnung an deren proximalen Ende und einem diese verbindendes Lumen (408), wobei das distale Ende der Röhre mit einem externen Schraubgewinde (403) mit gleichmäßiger Steigung konfiguriert ist, wobei die Röhre eine ausreichende Drehfestigkeit aufweist, um an dem proximalen Ende der Röhre aufgebrachte Drehkraft zu dem Gewinde zu übertragen,
**dadurch gekennzeichnet,**
**dass** das Gewinde eine Gewindehöhe von zumindest einer Hälfte (1/2) des äußeren Durchmessers der Katheterröhre aufweist und eine Gewindesteigung kleiner als der Umfang einer von dem Gewinde geformten Schraubenlinie.

**9.** Suprapubischer Gewindekatheter nach Anspruch 8,
**dadurch gekennzeichnet,**
**dass** das Gewinde (403) nicht länger als sechs Umdrehungen lang ist.

**10.** Suprapubischer Gewindekatheter nach Anspruch 8,
**dadurch gekennzeichnet,**
**dass** der Katheter äußerlich erfassbare Mittel zur Bestimmung der Ausrichtung des distalen Endes aufweist.

**11.** Gewindekatheter nach einem dcr Ansprüche 1 bis 4 und 8 bis 10,
**dadurch gekennzeichnet,**
**dass** die gegenüberliegenden Enden der Gewinde sich verjüngen von einer Gewindehöhe von zumindest einem Fünftel (1/5) des äußeren Durchmessers der Röhre auf die Gewindehöhe Null, um das Vorwärtskommen und das Einführen des Katheters in eine urogenitale oder gastrointestinale Passage des Säugers zu vereinfachen.

**12.** Mit Gewinde versehenes Kameraeinführungssystem zur Einsicht in den gastrointestinalen Trakt, umfassend eine flexible Katheterröhre (502) mit einem Lumen (508) und einem externen Gewinde (503) mit gleichmäßiger Steigung über die Länge der Röhre,
**dadurch gekennzeichnet,**
**dass** das System des Weiteren an dem distalen Ende der Röhre eine Bulbusspitze (501) umfasst, wobei das Lumen (508) sich von der Innenseite eines Fensters (511) an dem distalen Ende der Bulbusspitze zu dem proximalen Ende der Röhre erstreckt und dass das externe Gewinde (503) sich ebenfalls entlang der Spitze erstreckt und eine Gewindehöhe von zumindest einem Fünftel (1/5) des äußeren Durchmessers der Röhre entlang eines Abschnittes der Länge der Röhre aufweist und eine Gewindehöhe von weniger als einem Fünftel (1/5) des äußeren Durchmessers der Röhre entlang der Länge der Spitze.

13. Mit Gewinde versehenes Kameraeinführungssystem nach Anspruch 12,
 **dadurch gekennzeichnet,**
 **dass** das System des Weiteren eine an dem distalen Ende einer flexiblen hohlen Gliedersäule (531) angeordnete Kamera (520) umfasst, wobei sich ein Kabelbaum (541) von der Kamera (521) durch die Gliedersäule (531) und aus dessen proximalem Ende heraus erstreckt, wobei die Kamera (521) und die Gliedersäule (531) in die Röhre (502) einschiebbar und in dieser befestigbar sind, wobei die Kamera (521) konfiguriert ist, um durch das Fenster (511) zu schauen, wobei die Gliedersäule (531) eine ausreichende Drehfestigkeit aufweist, um an dem proximalen Ende der Gliedersäule aufgebrachte Drehkraft zu dem distalen Ende zu übertragen.

14. Mit Gewinde versehenes Kameraeinführungssystem nach Anspruch 12,
 **dadurch gekennzeichnet,**
 **dass** das System des Weiteren eine Trommel (551) mit einer axialen Öffnung (552) umfasst, um welche herum eine Handhabe (553) drehbar befestigt ist, wobei die Trommel (551) eine entsprechende Größe aufweist, um das Kameraeinführungssystem in einer Spulenkonfiguration zu halten, wobei die Trommel das System drehend durch die axiale Öffnung dispensiert, während die Handhabe in einem nicht drehenden Griff gehalten wird.

## Revendications

1. Cathéter fileté (101, 111, 121, 141) pour cathétériser les passages génito-urinaires et gastro-intestinaux de mammifères, ledit cathéter comprenant un tube flexible (102, 112, 122, 142) de cathéter ayant un passage (108, 128, 148) allant d'un orifice de drainage (107, 117, 127, 147) située sur l'extrémité distale (105, 115, 125, 145) jusqu'à l'extrémité proximale (106, 116, 126, 146), ladite extrémité distale dudit tube étant configurée avec un filet de vis extérieur (103, 113, 123, 143), de pas uniforme, ledit tube ayant une résistance à la torsion suffisante pour transmettre audit filet une force de rotation appliquée à ladite extrémité proximale dudit tube, *caractérisé en ce que* ledit filet possède une hauteur de filet égale à au moins un cinquième (1/5) du diamètre extérieur dudit tube de cathéter et un pas de vis inférieur à la circonférence de l'hélice formée par ledit filet.

2. Cathéter fileté selon la revendication 1, ledit filet (103, 113, 123, 143) n'étant pas supérieur à 6 spires.

3. Cathéter fileté selon la revendication 1, ledit tube (102, 112, 122, 142) de cathéter étant configuré pour accepter l'insertion dans ledit passage d'un stylet flexible (131) par ladite extrémité proximale (106, 116, 126, 146) dudit tube, la pointe distale (134) dudit stylet étant engageable dans un accessoire non rotatif fixé dans ledit passage (128) dudit cathéter à proximité de ladite extrémité distale, ledit stylet ayant une résistance à la torsion suffisante pour transmettre à ladite pointe dudit stylet une force de rotation appliquée à l'extrémité proximale dudit stylet.

4. Cathéter fileté selon la revendication 1, ledit cathéter (141) comprenant de plus un ballonnet gonflable (150) à paroi mince entourant et finissant sur ledit tube (142) à une extrémité de manière distale audit filet (143) et de manière proximale audit orifice de drainage (147), et à l'autre extrémité de manière proximale audit filet, un passage (151) destiné au gonflage s'étendant d'un orifice (152) de gonflage dans ledit tube à l'intérieur de l'enveloppe dudit ballonnet, jusqu'à ladite extrémité proximale dudit tube.

5. Dilatateur fileté (201) destiné à dilater les passages génito-urinaires et gastro-intestinaux de mammifères, ledit dilatateur comprenant un manche flexible (202) comportant un renflement effilé (204) à proximité de son extrémité distale, avec un filet hélicoïdal (203) de pas uniforme placé sur ce renflement, ledit manche (202) ayant une résistance à la torsion suffisante pour transmettre audit filet une force de rotation appliquée à ladite extrémité proximale dudit manche, *caractérisé en ce que* ledit filet possède une hauteur de filet égale à au moins un cinquième (1/5) du diamètre extérieur dudit manche et un pas de vis inférieur à la circonférence de l'hélice formée par ledit filet.

6. Endoprothèse (ou stent) filetée (301, 361) destinée à supporter les passages génito-urinaires et gastro-intestinaux de mammifères, ladite endoprothèse (301, 361) comprenant un tube flexible (302, 362) avec un filet hélicoïdal externe (303, 363) de pas uniforme placé sur celui-ci, ledit tube (302, 362) comprenant de plus des moyens pour un engagement axial selon une relation non rotative avec l'extrémité distale du stylet (331), *caractérisée en ce que* ledit filet possède une hauteur de filet égale à au moins un cinquième (1/5) du diamètre extérieur dudit tube et un pas de vis inférieur à la circonférence de l'hélice formée par ledit filet.

7. Endoprothèse filetée selon la revendication 6, ladite endoprothèse (361) comprenant de plus des moyens de contrôle du flux, lesdits moyens étant un clapet de non-retour (371) placé à l'intérieur de ladite endoprothèse, ledit clapet de non-retour (371) étant actionnable par utilisation d'un stylet (331).

8. Cathéter sus-pubien fileté (401) pour le drainage d'un organe génito-urinaire, ledit cathéter comprenant un tube (402) de cathéter flexible ayant un premier orifice axial à son extrémité distale et un second orifice axial à son extrémité proximale et un passage (408) de communication entre ceux-ci, ladite extrémité distale dudit tube étant configurée avec un filet de vis extérieur (403) de pas uniforme, ledit tube ayant une résistance à la torsion suffisante pour transmettre audit filet une force de rotation appliquée à ladite extrémité proximale dudit tube, ***caractérisé en ce que*** ledit filet possède une hauteur de filet égale à au moins une moitié (1/2) du diamètre extérieur dudit tube de cathéter et un pas de vis inférieur à la circonférence de l'hélice formée par ledit filet

9. Cathéter sus-pubien fileté selon la revendication 8, ledit filet (403) étant d'une longueur non supérieur à six spires.

10. Cathéter sus-pubien fileté selon la revendication 8, ledit cathéter ayant des moyens détectables extérieurement pour déterminer l'orientation de ladite extrémité distale.

11. Cathéter fileté selon l'une quelconque des revendications 1 à 4 et 8 à 10, dans lequel les extrémités opposées dudit filet sont effilées d'une hauteur de filet égale à au moins un cinquième (1/5) du diamètre extérieur dudit tube à une hauteur zéro afin de faciliter l'avancée et le recul du cathéter dans un passage génito-urinaire ou gastro-instestinal de mammifcres.

12. Système fileté d'introduction d'une caméra pour accéder au trajet gastro-intestinal, comprenant un tube (502) de cathéter flexible avec un passage (508) et un filet externe (503) de pas uniforme sur la longueur dudit tube, ***caractérisé en ce que*** le système comprend en plus une pointe renflée (501) à l'extrémité distale dudit tube, ledit passage (508) s'étendant de la face intérieure d'une fenêtre (511) sur l'extrémité distale de ladite pointe renflée jusqu'à l'extrémité proximale dudit tube, et ***en ce que*** ledit filet externe (503) s'étend aussi le long de ladite pointe et possède une hauteur de filet égale à au moins un cinquième (1/5) du diamètre extérieur dudit tube sur une portion de la longueur dudit tube et une hauteur de filet inférieure à un cinquième (1/5) du diamètre extérieur dudit tube sur la longueur de ladite pointe.

13. Système fileté d'introduction d'une caméra selon la revendication 12, ledit système comprenant en plus une caméra (520) fixée sur l'extrémité distale d'un bras vertébral creux flexible (531), un harnais (541) de câbles allant de ladite caméra (521), via ledit bras vertébral (531) et à l'extérieur de l'extrémité proximale de celui-ci, ladite caméra (521) et ledit bras vertébral (531) étant insérables et fixables à l'intérieur dudit tube (502), ladite caméra (521) étant configurée pour voir à travers ladite fenêtre (511), ledit bras vertébral (531) ayant une résistance suffisante à la torsion pour transmettre à ladite extrémité distale une force de rotation appliquée à ladite extrémité proximale dudit bras vertébral.

14. Système fileté d'introduction d'une caméra selon la revendication 12, comprenant en plus un tambour (551) avec une ouverture axiale (552) autour duquel une poignée (553) est fixée en rotation, ledit tambour (551) étant dimensionné pour maintenir ledit système fileté d'introduction d'une caméra dans une configuration enroulée, ledit tambour distribuant relativement ledit système par ladite ouverture axiale pendant que ladite poignée est maintenue selon une prise non rotative.

FIG. 1

105

103

101 102

106

104

FIG. 2

002

002

103

107

107

101

102

108

FIG. 4

103b 103a

107 102

102d 103d

103

103c

FIG. 3

FIG. 7

FIG. 5

FIG. 6

125 124 123 127 128

FIG. 8

142 148 151

FIG. 11

134 132

FIG. 9

150 153 141 149 142 148 143 152 151 146b 146a

FIG. 10

FIG. 12

FIG. 13

FIG. 14

FIG. 15

FIG. 16

FIG. 17

FIG. 18

334

331

332

333

**FIG. 19**

345

343

342

341

346

**FIG. 21**

334

332

331

**FIG. 20**

354

352

341

351

342

353

343

347

**FIG. 22**

FIG. 23

FIG. 24

FIG. 25

EP 0 957 966 B1

FIG. 26

FIG. 27

FIG. 28

FIG. 29

29

_500_

503

502

511

508

501

503c

502d

FIG. 31A

_500_

503

502

FIG. 31B

511

501

_500_

503

502

508

FIG. 30

520

531

532

521

523 522

541

541

FIG. 32

512

521

532

511

501 508 503 502 541

FIG. 33

550

551

552 553

FIG. 34